(19) Europäisches Patentamt
European Patent Office
Office européen des brevets

(11) EP 0 614 911 B1

(12) # FASCICULE DE BREVET EUROPEEN

(45) Date de publication et mention de la délivrance du brevet:
**28.04.1999 Bulletin 1999/17**

(51) Int. Cl.[6]: **C07K 5/06**, A61K 38/05

(21) Numéro de dépôt: **94400331.8**

(22) Date de dépôt: **15.02.1994**

(54) **Composés à groupe sulfamoyle et amidino, leur procédé de préparation et les compositions pharmaceutiques les contenant**

Verbindungen mit eine Sulfonamid- und einer Amidinogruppe, ihr Herstellungsverfahren und sie enthaltende pharmazeutische Zubereitungen

Compounds having a sulfonamide and an amidinegroup, process for preparation and pharmaceutical compositions comprising them

(84) Etats contractants désignés:
**AT BE CH DE DK ES FR GB GR IE IT LI LU MC NL PT SE**

(30) Priorité: **15.02.1993 FR 9301686**

(43) Date de publication de la demande:
**14.09.1994 Bulletin 1994/37**

(73) Titulaire: **SANOFI**
**75008 Paris (FR)**

(72) Inventeurs:
- **Christophe, Bernard**
  **B-1367 Grand Rosière (BE)**
- **Foulon, Loic**
  **F-31120 Pinsaguel (FR)**
- **Pellet, Alain**
  **F-31870 Beaumont sur Leze (FR)**
- **Serradeil-Le-Gal, Claudine**
  **F-31750 Escalquens (FR)**
- **Valette, Gérard**
  **F-31120 Lacroix Falfarde (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
**EP-A- 0 236 163**
**WO-A-91/08223**
**WO-A-92/16549**

- **NEUROSCIENCE LETTERS vol. 124, no. 1 , 11 Mars 1991 pages 1 - 4 C. LAURENS ET AL. 'Enhancement of neurite outgrowth from central nervous system neurons in primary culture by thrombin inhibitors'**
- **DRUGS OF THE FUTURE vol. 17, no. 1 , 1992 pages 39 - 45 M.C. MICHEL AND A. BUSCHAUER 'Neuropeptide Y and its antagonists'**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux composés comportant simultanément notamment un groupe sulfamoyle substitué et un groupe amidino, leur procédé de préparation et les compositions pharmaceutiques les contenant.

Ces composés ont en particulier une certaine affinité pour les récepteurs biologiques du neuropeptide Y, NPY, présents dans les systèmes nerveux central et périphérique.

Le neuropeptide Y n'a été mis en évidence qu'il y a une dizaine d'années, et on connaît actuellement très peu d'agonistes ou d'antagonistes de ses récepteurs qui ne soient pas des polypeptides dont l'utilisation en thérapeutique n'est pas simple, notamment à cause de leur dégradation dans le tractus gastro-intestinal; une revue récente dans Drugs of the Future 17 (1) 39-45 (1992) cite la benextramine, un phosphate d'inositol et un antihistaminique dérivé de guanidinoalkyl-imidazole.

Des composés de structure proche de celle des composés de l'invention ont été décrits dans EP-A-0 236 163 et EP-A- 0 236 164; ils répondent à la formule A :

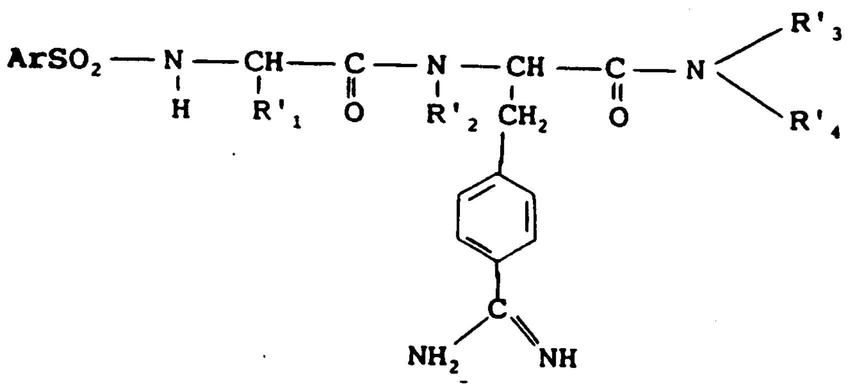

(A)

dans laquelle $R'_1$, $R'_2$, $R'_3$, $R'_4$ sont notamment des alkyles ou des phényles. Ces composés sont des anticoagulants et des antithrombotiques, de telle sorte que ce document ne pouvait pas suggérer l'activité des présents composés.

Les composés de l'invention répondent à la formule I :

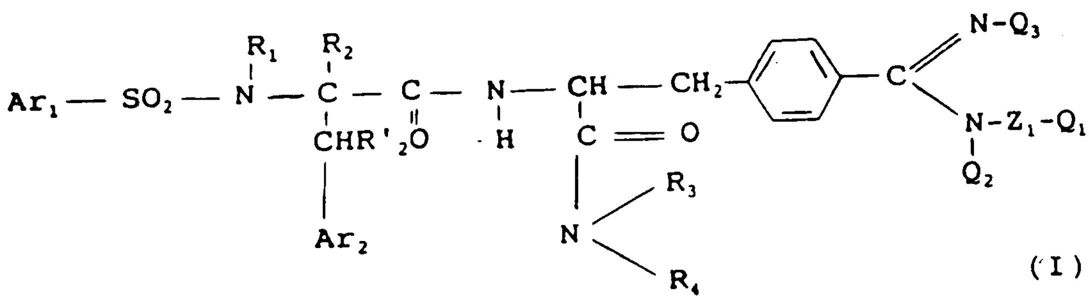

(I)

dans laquelle

$Ar_1$ représente naphtyle, phényle, quinolyle ou isoquinolyle éventuellement substitués par Cl, F, ($C_1$-$C_4$)alkyle, ($C_1$-$C_4$)alkoxy, hydroxyle ou ($C_1$-$C_4$)dialkylamino;
$Ar_2$ représente phényle ou thiényle éventuellement substitués, par Cl, F, ($C_1$-$C_4$)alkyle, ($C_1$-$C_4$)alkoxy ou hydroxyle;
$R_1$, $R_2$ et $R'_2$ représentent indépendamment l'un de l'autre H ou alkyle;
ou $R_1$ ne représente rien et N est lié à $Ar_2$, et éventuellement $R_2$ et $R'_2$ forment une double liaison ;
ou $R_1$ ou $R_2$ est lié à $Ar_2$ et représente alkylène en ($C_1$, $C_2$ ou $C_3$);
$R_3$ et $R_4$ identiques ou différents, représentent H, alkyle ou forment avec l'atome d'azote auquel ils sont liés un hétérocycle saturé en $C_5$ à $C_7$ choisi parmi pyrrolidine, pipéridine et hexahydroazépine ;
$Z_1$ représente alkylène en $C_1$ à $C_{12}$, interrompu ou prolongé éventuellement par cycloalkyle en $C_5$ à $C_7$ ou phényle;
$Q_1$ représente méthyle, phényle éventuellement substitué, amino, alkoxycarbonylamino, alkylamino, dialkylamino,

un groupe amino hétérocyclique saturé en $C_5$ à $C_7$, amidino, alkylamidino, guanidino, alkylguanidino, pyridyle, imidazolyle, pyrimidinyle, indolyle, hydroxy, alkoxy, alkoxycarbonyle en $C_2$ à $C_8$,amino ($C_1$-$C_4$)alkyl-N-($C_1$-$C_4$)alkylamino ou carbamoyle;

$Q_2$ représente H ou alkyle;

$Q_3$ représente H ou ($C_1$-$C_4$)alkyle; ou $Q_1$ et $Q_3$ sont liés pour former un hétérocycle et représentent ensemble un alkylène en $C_2$ ou $C_3$, tandis que

$Z_1$ ne représente rien,

et les sels d'addition de ces composés avec des acides.

Les groupes alkyles et alkoxy, linéaires ou ramifiés sont en $C_1$ à $C_4$ sauf mention contraire ; les groupes amino hétérocycliques saturés peuvent être pyrrolidinyle, pipéridino, morpholino, pipérazinyle, alkyl-4 pipérazinyle. Les phényles, sauf mention contraire, peuvent être substitués par Cl, F, alkyle en $C_1$ à $C_4$ ou alcoxy en $C_1$ à $C_4$, un hydroxyle.

Les sels sont en général préparés avec des acides pharmaceutiquement acceptables mais les sels d'autres acides utiles à la purification ou l'isolement de produits de formule I font aussi partie de l'invention.

Les composés de formule I comprennent en général 2 carbones asymétriques et les 4 énantiomères purs ainsi que leurs mélanges en proportions quelconques sont objets de l'invention.

Les composés selon l'invention peuvent être préparés à partir des composés de formule II :

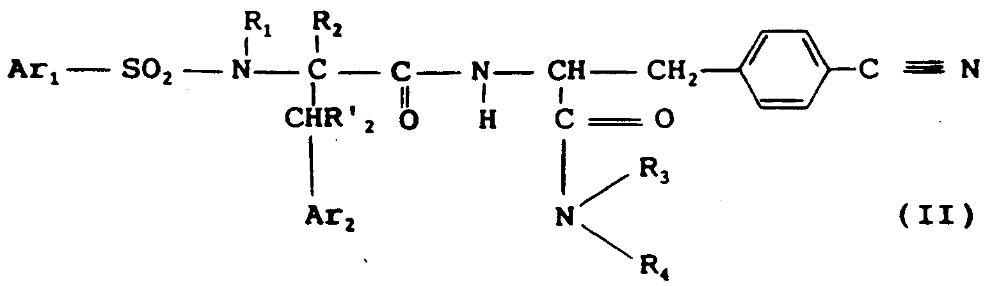

(II)

par des procédés dont les principes sont connus, que l'homme du métier sera à même d'adapter à la réactivité et à la solubilité des produits mis en oeuvre.

De nombreux procédés de synthèse des amidines sont décrits dans l'ouvrage "The chemistry of amidines and imidates" D.G. Neilson Ed Saul Patai; Wiley & Sons - p. 389-394 (1975). En général le nitrile est transformé en sel d'imidate par action d'un alcool en milieu acide fort, dans une réaction dite de Pinner, et cet imidoester, éventuellement sous forme libre, est mis à réagir avec l'amine de formule III :

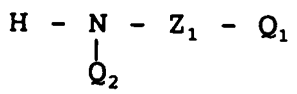

III

dans un solvant polaire non réactif, de préférence dans un alcool, à une température comprise entre 0°C et la température de reflux du solvant.

La plupart de ces amines (III) sont connues et les produits nouveaux peuvent être préparés en appliquant des principes et méthodes bien connus de l'homme du métier. Par exemple, pour les dérivés dans lesquels $Q_1$ est un imidazolyle, on se réfèrera à US-3,881,016 et à Synth. Communic. 17, 223-227 (1987) ou lorsque $Q_1$ est un groupe t-butoxycarbonylamino à Synth. Communic. 20 (16), 2559-2564 (1990).

Les composés de formule I dans laquelle $Q_1$ représente $NH_2$ ou alkylamino peuvent être préparés par hydrolyse des composés de formule I dans laquelle $Q_1$ est un groupe t-butoxy-carbonylamino.

On peut préparer les composés de formule I dans lesquels $Q_1$ représente un groupe guanidino, substitué ou non, par action sur le composé dans lequel $Q_1$ = $NH_2$, d'un composé de formule

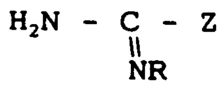

dans lequel R représente H ou alkyle et Z représente un nucléofuge, tel que $SO_3H$, par exemple dans les conditions décrites dans Tetrahedron Letters 3183-3186 (1988) avec l'acide aminoimino méthane sulfonique ; l'acide N-méthylamino iminométhanesulfonique peut être obtenu comme décrit dans J. Org. Chem. 51 1882 (1986).

Les composés de formule I dans lesquels la fonction amidine est comprise dans un hétérocycle peuvent être préparés de façon connue en soi par action d'une diamine $H_2N\text{-}(CH_2)_n\text{-}NH_2$ dans laquelle n est 2 ou 3 sur l'imidoester, éventuellement par action d'une diamine dont l'une des fonctions est protégée par un groupement labile qui sera éliminé avant la cyclisation.

Un certain nombre de procédés de préparation des nitriles de formule II dans laquelle $Ar_1$ est naphtyle, $R_1$=$R_2$ = $R'_2$=H sont décrits dans EP-A-0 236 163 et on pourra s'y référer, notamment pour préparer les énantiomères purs à partir de chaque stéréoisomère pur de la 4-cyanophénylalanine dont la fonction acide carboxylique sera bloquée, éventuellement, sous forme d'amide substituée par $R_3$ et $R_4$ comme dans la formule I ; on fera réagir sur ce composé l'alpha-aminoacide de formule IV:

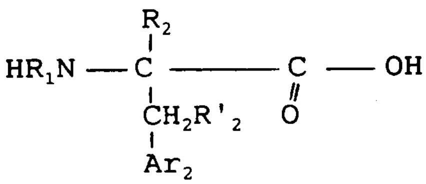

IV

dont le groupe amino sera préalablement protégé soit sous forme de sulfamoyle $Ar_1\text{-}SO_2\text{-}N\langle$ comme dans la formule I,soit par un groupe labile, tel que t-butoxycarbonyle, qui sera éliminé après la condensation, de façon classique par action d'un acide fort anhydre.

On trouve aussi dans les nombreuses publications concernant la chimie des peptides et notamment dans : The Peptides. Ed. E. Cross et J. Meienhofer vol. 1, 65-104 (1979) - Acad. Press., des procédés de préparation d'amides par réaction d'un groupe carboxylique et d'un groupe amino, portés par 2 carbones asymétriques, sans racémisation autour de l'un quelconque de ces carbones.

En général, ces réactions ont lieu à des températures comprises entre 0 et 40°C, dans un solvant inerte tel que dichlorométhane, acétonitrile, tétrahydrofuranne ou diméthylformamide, en présence d'au moins un équivalent d'une amine tertiaire telle que la triéthylamine ou de préférence, en présence de N-éthylmorpholine.

Le groupe sulfamoyle

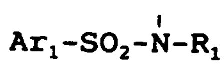

peut être obtenu de façon classique, par action d'un sulfo-chlorure $Ar_1\text{-}SO_2\text{-}Cl$ en présence d'une base, éventuellement en milieu biphasique en présence d'un catalyseur de transfert de phase, soit sur l'aminoacide IV ou un ester d'alkyle correspondant, soit sur le nitrile de formule V :

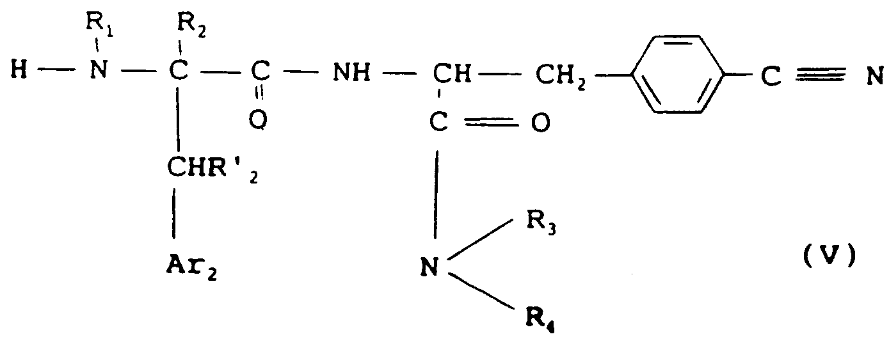

(V)

Lorsque $R_1$ est différent de H, les nitriles II peuvent être obtenus par action de $R_1X$ sur le sulfonamide II dans lequel $R_1$ est H, en présence d'une base, X représentant un atome d'halogène ou un groupe sulfonate.

Les alpha-aminoacides de formule IV ou les esters aliphatiques correspondants sont des composés connus ou peuvent être préparés par des procédés analogues à ceux utilisés pour les dérivés connus. On peut se référer notamment à J.P. Greenstenin et M. Winitz dans Chemistry of the Amino Acids; J. Willey and Sons, Inc. ed, 1961, p. 697 à 714, p. 2693 à 2770, à G.C. Barrett dans Chemistry and Biochemistry of the Amino Acids, Chapman and Hall Ltd ed., 1985, p. 246 à 353.

Par exemple, lorsque $R'_2$ = H par l'intermédiaire d'une base de Schiff comme décrit dans Synthesis 313-315 (1984), selon le schéma réactionnel :

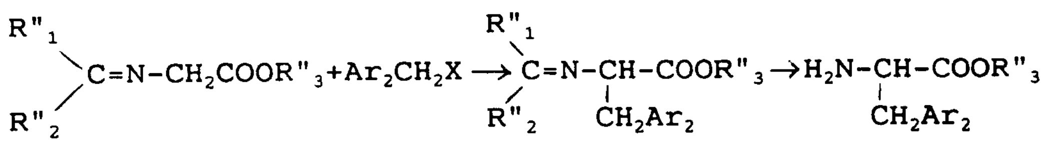

dans lequel $R''_1$ et $R''_2$, représentent des noyaux phényles, $R''_3$ représente alkyle et $Ar_2$ a la même signification que dans la formule I.

On peut obtenir les alpha-aminoacides de formule IV dans lesquels $R_2$ est différent de H par une variante de la méthode précédente. La base de Schiff est alkylée successivement par $Ar_2CH_2X$ puis par $R_2X$ en opérant dans le THF avec une base telle qu'un alcoolate alcalin entre -70° et 25°C.

Pour obtenir les aminoacides de formule IV, ou les esters correspondants sous forme de l'un des énantiomères pur, on peut effectuer des recristallisations fractionnées d'un sel du racémique avec un acide ou une base optiquement active, selon une technique dont le principe est bien connu; on peut aussi séparer l'un des énantiomères d'un ester racémique d'un aminoacide de formule IV sous forme de l'aminoacide correspondant, en effectuant une hydrolyse enzymatique du mélange racémique avec une enzyme stéréosélective telle que l'alpha-chymotrypsine, méthode décrite notamment dans Journal of Biochemistry 19, 877-881 (1971).

Les sels des composés de formule I sont préparés par action des acides choisis, sur l'amidine de formule I, dans un solvant; les sels obtenus sont isolés après distillation du solvant ou addition d'un non solvant pour les précipiter.

Les composés de formule I et leurs sels pharmaceutiquement acceptables se fixent sur les récepteurs biologiques du neuropeptide Y, (NPY), peptide de 36 acides aminés dont les activités physiologiques sont multiples, notamment dans le système nerveux central ou cardiovasculaire. Le NPY contrôle l'activité psychomotrice, l'anxiété, la sédation, c'est un stimulant de la prise de nourriture; il intervient dans la dépression, les processus de mémorisation, certains comportements sexuels et l'épilepsie; il inhibe la sécrétion d'insuline, de glucagon et d'hormone lutéïnisante; il agit au niveau du rein et notamment sur le système rénine-angiotensine; enfin, c'est un vasoconstricteur puissant. On peut se référer à une revue publiée dans Drugs of the Future 17 (1) 39-45 (1992) qui mentionne aussi des activités thérapeutiques potentielles des antagonistes du NPY.

L'affinité des composés de l'invention pour les récepteurs du NPY peut être mise en évidence, in vitro, en utilisant la méthode décrite par Unden et Coll. dans Eur. J. Biochem 145 525-530 (1984) sur des membranes de cortex

de rat; dans ces conditions, les composés de l'invention exemplifiés dans ce qui suit ont des $CI_{50}$ (concentration inhibitrice de 50% de la liaison du NPY à son récepteur) comprises entre 10 nM et 10 µM, tandis que celle du NPY est de 0,5 nM.

Les composés affins peuvent être agonistes ou antagonistes de l'action du peptide NPY sur son récepteur.

L'activité antagoniste du NPY peut être démontrée en appliquant la méthode décrite dans Proc. Soc. Exp. Biol. Med. 64 453-455 (1947) chez le rat amyélé; dans ces conditions, l'administration de NPY a un effet hypertenseur qui est diminué voire supprimé lorsque les animaux sont traités par un antagoniste de l'invention.

Pour les composés ayant une forte affinité pour les récepteurs, on a mesuré des $DI_{50}$ de quelques µg/kg lors de perfusions i.v. de 10 µg/kg de NPY.

Actuellement on ne connaît pas d'antagoniste spécifique, de grande affinité et compétitif et les composés selon l'invention sont particulièrement intéressants ; ils pourront être utilisés avantageusement comme antihypertenseurs ou pour le traitement de l'angine de poitrine notamment pour leur activité vasodilatatrice, ou pour lutter contre les vasospasmes coronaires et cérébraux, ainsi que dans le traitement de l'athérosclérose et de l'insuffisance cardiaque. Ces composés pourront aussi être utiles comme agents anorexigènes, antidépresseurs, tranquilisants, pour diminuer l'anxiété ou réguler certains troubles du comportement sexuel. Ils présenteront aussi un réel intérêt dans le traitement de l'inflammation, de l'allergie, de certains désordres gastro-intestinaux, tels que la maladie de Crohn ou le contrôle de la prise de nourriture, ou encore dans celui des surcharges graisseuses, étant donné leur activité lipolytique ; ce sont aussi des immunomodulateurs. Ils pourront être utilisés dans toutes les pathologies ou désordres NPY dépendants.

Ainsi, l'invention concerne aussi les compositions pharmaceutiques comprenant comme principe actif l'un des énantiomères des composés de formule I, l'un de leur mélange ou les sels de ceux-ci avec un acide pharmaceutiquement acceptable, ainsi qu'un excipient convenable pour une administration par voie orale, injectable ou transdermique. Les doses journalières seront fonction de la pathologie à traiter et du malade.

La présente invention a également pour objet des compositions pharmaceutiques contenant une dose efficace d'un composé selon l'invention ou d'un sel pharmaceutiquement acceptable et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intraveineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'adminsitration sous-cutanée, intramusculaire ou intraveineuse et les formes d'administration rectale. Pour l'appplication topique, on peut utiliser les composés selon l'invention dans des crèmes, pommades ou lotions.

Afin d'obtenir l'effet prophylactique ou thérapeutique désiré, la dose de principe actif peut varier entre (0,01 et 50 mg) par kg de poids du corps et par jour.

Chaque dose unitaire peut contenir de 0,5 à 1000 mg de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température, rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un des sels pharmaceutiquement acceptables, d'autres principes actifs qui peuvent être utiles dans le traitement des troubles ou maladies indiquées ci-dessus.

On préfère particulièrement les composés dans lesquels $Z_1$ représente alkylène en $C_4$ à $C_9$ et $Q_1$ est fixé sur $Z_1$ par un atome d'azote et représente un groupe amino, guanidino ou amidino, substitués ou non; d'autre part, on préfère les composés dans lesquels $NR_3R_4$ représente pyrrolidinyle.

On préfère tout particulièrement les composés (I) dans lesquels $Z_1$ représente méthylènecyclohexylméthylène, $Q_1$ représente amino, alkylamino, dialkilamino, $R_3$ et $R_4$ constituent avec l'azote auquel ils sont liés un pyrrolidinyle, $Ar_2$ représente un phényle ou méthoxyphényle, $Ar_1$ représente un naphtyle et $Q_2$, $Q_3$, $R_1$, $R_2$ et $R'_2$ sont tels que définis pour (I).

Dans ce qui suit, on décrit des exemples de composés de l'invention et de procédés de préparation. Préalablement, la préparation d'un certain nombre de composés intermédiaires de synthèse, est indiquée, à titre d'illustration.

Les composés de formule I comportent en général deux carbones asymétriques et peuvent être isolés sous forme d'un mélange de deux couples de racémiques diastéréoisomères, dont les proportions relatives dépendront des conditions opératoires, étant donné leurs propriétés physiques différentes. Lorsque les produits de départ qui comportent un carbone asymétrique ne sont pas des mélanges racémiques mais sont enrichis en l'un ou l'autre des énantiomères, le produit final ne sera pas en général un mélange de deux racémiques sauf si les conditions opératoires entraînent la racémisation.

Dans les produits de formule I décrits dans ce qui suit, on a mesuré les proportions relatives des deux couples de racémiques par des méthodes classiques, telle que la chromatographie liquide haute performance ou la spectroscopie de résonance magnétique nucléaire.

Sauf indication contraire, les nitriles de formule II isolés sont un mélange équimoléculaire de diastéréoisomères.

**A - <u>Préparation de sulfonamides</u>**

**. Acide N-(2-naphtyl sulfonyl)1,2,3,4-tétrahydroisoquinoléine 3-carboxylique.**

On introduit dans une suspension de 5 g de chlorhydrate de l'acide 1,2,3,4-tétrahydroisoquinoléine-3-carboxylique dans 150 ml de dioxanne, 46,8 ml d'une solution aqueuse de NaOH N puis lentement 5,3 g de chlorure de 2-naphtalènesulfonyle et une solution de NaOH N pour maintenir un pH voisin de 10 - Après la fin de l'addition, le mélange est maintenu 3 heures sous agitation vers 20°C avant l'addition de 150 ml de $CH_2Cl_2$. Après acidification de la phase aqueuse jusqu'à pH 2, on sépare la phase organique et réextrait la phase aqueuse par 150 ml de $CH_2Cl_2$. Les phases organiques sont séchées, concentrées et le résidu est purifié par chromatographie sur gel de silice, en éluant avec un mélange de chlorure de méthylène et de méthanol (80/20-V/V). On obtient 7 g de sulfonamide sous forme d'un hémihydrate F = 110°C.

. N-(3,4-dichlorophényl sulfonyl) O-méthyl tyrosine.

On introduit sous agitation 5 g de chlorure de 3,4-dichlorophénylsulfonyle dans un mélange de 3,8 g de O-méthyl-tyrosinate d'éthyle dans 35 ml de $CH_2Cl_2$ et 50 ml d'une solution aqueuse saturée de carbonate de potassium. Après une nuit, le solide est éliminé, la phase organique séparée et la phase aqueuse réextraite avec $CH_2Cl_2$. Les phases organiques séchées sont concentrées et le résidu est chromatographié sur une colonne de silice en éluant avec un mélange de $CH_2Cl_2$ et $CH_3OH$ (95/5-V/V). On obtient 6,8 g de N-(3,4-dichlorophénylsulfonyl) O-méthyltyrosinate d'éthyle, racémique qui fond à 99°C. Cet ester est hydrolysé dans 100 ml de $C_2H_5OH$ contenant 9,5 ml d'une solution aqueuse de KOH 5N pour donner après acidification 5,5 g de l'acide correspondant qui fond à 183°C.

De la même manière, on prépare les :

. N-(2-naphtyl sulfonyl) phénylalanine
  F=146°C (ester méthylique : F = 144°C)

. N-(2-naphtyl sulfonyl) O-méthyl tyrosine
  F=174°C (ester éthylique : F = 138°C)

. N-méthyl N-(2-naphtyl sulfonyl) phénylalanine
  F=122°C (ester méthylique : F = 106°C)

. Acide N-(2-naphtyl sulfonyl)2-amino indane-2 carboxylique

F = 264°C

. N-(5-isoquinolyl sulfonyl)alpha-méthyl phenylalanine
(Ester éthylique : F = 60°C)

. N-(8-quinolinyl sulfonyl)O-méthyl tyrosine
F=228°C

. N-(2-naphtyl sulfonyl)O-benzyl tyrosine
F=182°C

. N-(1-naphtyl sulfonyl)2,4-diméthyl phénylalanine
F=220°C
(ester éthylique : F=134°C)

. N-(4-tolyl sulfonyl)4-chorophénylalanine
F=164°C
(ester éthylique F=114°C)

Le 2-amino indane-2 carboxylate d'éthyle de départ peut être préparé à partir du N-diphénylméthylène glycinate d'éthyle : à - 70°C, on introduit 25 g de N-diphénylméthylène glycinate d'éthyle dans 1500 ml de tétrahydrofuranne contenant 10,5 g de tertiobutylate de potassium, puis lentement 12,6 ml d'$\alpha,\alpha$-(dibromo) orthoxylène et après 12 heures, 10,5 g de tertiobutylate de potassium. On laisse revenir à température ambiante et après 12 heures, on introduit dans le milieu une solution aqueuse saturée de $NH_4Cl$. Les solvants sont éliminés par distillation sous pression réduite et le résidu extrait par $(C_2H_5)_2O$. La phase organique séparée est agitée durant 16 heures, à température ambiante avec 150 ml d'une solution aqueuse de HCl N. La solution aqueuse, après 3 lavages avec $(C_2H_5)_2O$ est amenée à pH 8 par addition de $NaHCO_3$ et 13,4 g d'ester recherché en sont extraits dans $CH_2Cl_2$.
F = 56°C.

**B. <u>Préparation de (4-cyano)phenyl-alanylamides</u>**

**1. Ester éthylique de (4-cyano)phénylalanine.**

Dans une solution de 40,4 g de 4-(bromométhyl)benzonitrile dans 460 ml de $CH_3CN$ anhydre, on introduit 7,23 g de bromure de tétrabutylammonium, 93 g de $Na_2CO_3$ puis 60 g de N-(diphénylméthylène)glycinate d'éthyle. Le milieu est maintenu 4 heures à sa température de reflux puis les solides sont séparés et les solvants éliminés par distillation sous pression réduite. Le résidu est repris par 1 litre de $(C_2H_5)_2O$ puis, après filtration, concentré jusqu'à 500 ml avant d'y ajouter 300 ml d'une solution aqueuse de HCl N. Après 16 heures d'agitation le mélange est décanté, et le pH de la phase aqueuse séparée est amenée vers 8. Le produit final en est extrait dans $CH_2Cl_2$. On obtient 34,8 g d'ester sous forme huileuse dont le chlorhydrate fond à 170°C.

**2. Séparation des isomères lévogyre et dextrogyre de l'ester précédent par hydrolyse enzymati-que.**

On agite durant 16 heures vers 25°C, 10 g de l'ester racémique, 20 mg d'alpha-chymotrypsine et 0,9 g d'albumine sérique bovine dans 1 l d'une solution aqueuse de $CH_3COONa$ 0,1M dont le pH est amené entre 6,5 et 6,8 par addition d'une solution aqueuse de NaOH 0,1N. Après filtration du milieu sur du talc puis du charbon actif, la moitié du solvant est éliminée par distillation sous pression réduite vers 35°C et la solution aqueuse restante amenée à pH 8 par addition de NaOH, puis extraite par $CH_2Cl_2$. Après les traitements habituels de la phase organique, on obtient 4,5 g de 2-amino 3-(4-cyanophényl) propionate d'éthyle, lévogyre, huileux
$[a]_D^{20}$ = - 27° (C=1;$CH_3OH$)

La solution aqueuse basique contient le sel de sodium de l'acide correspondant à l'autre énantiomère. Après acidification jusqu'à pH 4 puis lyophilisation, on isole une poudre blanche qui contient l'aminoacide lévogyre.

**3. N-(t-butoxycarbonyl) 4-cyanophénylalanine.**

On introduit à 5°C, 20 ml de solution aqueuse de NaOH N et 4,34 g de di-(tertiobutyl)carbonate dans une solution de 4,34 g d'un ester éthylique de 4-cyanophénylalanine dans 70 ml de dioxanne. Après retour à la température ambiante et 3 heures sous agitation, le milieu réactionnel est amené à sec; on verse ensuite 100 ml d'eau sur le résidu et après un lavage avec $CH_3COOC_2H_5$, on amène la solution aqueuse à pH 2 par addition d'une solution de $KHSO_4$; on extrait ensuite le produit final dans $CH_2Cl_2$.

**4. 1-[2-amino 3-(4-cyanophényl)] propionyl pyrrolidine.**

A 0°C, on introduit 1,98 g de pyrrolidine et 3 g d'hydroxybenzotriazole dans 70 ml de $CH_2Cl_2$ contenant 5,4 g de N-(t-butoxycarbonyl) 4-cyanophénylalanine, puis vers -5°C une solution de 4 g de dicyclohexylcarbodiimide dans 30 ml de $CH_2Cl_2$. Après 16 heures d'agitation à 20°C, on filtre et lave la phase organique par une solution aqueuse saturée de $Na_2CO_3$, puis par une solution de $KHSO_4$ de pH 2 et enfin avec de l'eau. Après les traitements habituels, on obtient 4,73 g du dérivé recherché dont la fonction amine primaire est protégée par un groupement t-butoxycarbonyle; ce groupement peut être éliminé par action d'un acide : on dissout le composé dans 50 ml d'acétate d'éthyle et on ajoute vers 0°C, 50 ml d'une solution d'acétate d'éthyle saturée à 15°C en HCl ; après 2 heures d'agitation vers 20°C, le solvant est éliminé et on obtient le chlorhydrate du produit cherché.

Le chlorhydrate racémique fond à 224°C.

L'énantiomère, préparé à partir de l'ester lévogyre, est lévogyre :

$[\alpha]^{20}_{D}$ = - 68°8 (C=1; $CH_3OH$)

tandis que celui préparé à partir de l'aminoacide résiduel lévogyre est dextrogyre, dans les mêmes condition de mesure.

**5. 1-[2-amino 3-(4-cyanophényl)]propionyl pipéridine.**

Le chlorhydrate racémique fond à 218°C; le composé intermédiaire N-(t-butoxycarbonyl) fond à 132°C.

**6. [N-méthyl N-éthyl] 2-amino 3-(4-cyanophényl) propionamide.**

Le chlorhydrate racémique fond à 228°C.

**C. <u>Préparation de composés de formule II</u>**

**1. Par réaction d'un sulfonamide et d'un (4- cyano)phénylalanylamide.**

. 1-(2-[2-(3,4-dichlorophénylsulfamoyle) 3-(4-méthoxyphényl) propionamido] 3-(4-cyanophényl)propionyl)pyrrolidine (composé 1)

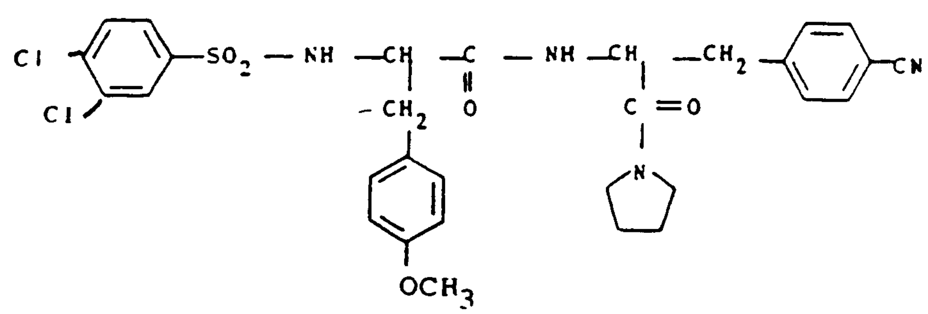

Dans 100 ml d'acétonitrile, à 0°C, on introduit 2,5 g de N-(3,4-dichlorophénylsulfonyl) O-méthyltyrosine, 1,82 g de chlorhydrate de 1-[2-amino 3-(4-cyano)phénylpropionyl]pyrrolidine et 2,87 g d'hexafluorophosphate de benzotriazolyl-1 oxytris (diméthylamino)phosphonium (BOP) puis 1,75 ml de triéthylamine à une température inférieure à 5°C. Après 16 heures d'agitation à température ambiante vers 20°C, on élimine le solvant sous pression réduite, et on dissout le résidu dans 80 ml de $CH_3COOC_2H_5$. Après lavage de la phase organique par une solution aqueuse à pH 2, par une solution saturée de $NaHCO_3$ puis à l'eau, le solvant est éliminé par distillation et le résidu est chromatographié sur une colonne de gel de silice en éluant avec un mélange $CH_2Cl_2/CH_3OH$ (98/2-V/V). On obtient 2,9 g d'un mélange de diastéréoisomères du nitrile qui fond à 101°C.

De la même manière on obtient :

. N-éthyl-N-méthyl-2-[2-(3,4-dichlorophénylsulfamoyl) 3-(4-méthoxyphényl)propionamido] 3-(4-cyanophényl)propionamide (composé 2).

F = 192°C, cristallisé avec 1,5 $H_2O$

. 1-(2-[2-(2-naphtylsulfamoyl) 3-(4-méthoxyphényl)propionamido]3-(4-cyanophényl)propionyl)pyrrolidine (composé 3)

Préparée à partir du sulfonamide racémique et du chlorhydrate de 1-[2-amino 3-(4-cyanophényl)-propionyl]pyrrolidine, lévogyre.

F = 135°C, cristallisé avec 1,5 $H_2O$

. 1-(2-[2-(2-naphtylsulfamoyl) 3-phényl propionamido] 3-(4-cyanophényl)propionyl)pyrrolidine (composé 4)

F = 206°C, cristallisé avec $H_2O$

. 1-(2-[2-(2-naphtylsulfamoyl) 3-phényl propionamido] 3-(4-cyanophényl)propionyl)pipéridine (composé 5)

F = 210°C, cristallisé avec $H_2O$

. N-éthyl-N-méthyl-2-[2-(1 naphtylsulfamoyl) 3-(3,4-dichlorophényl)propionamido]-3-(4-cyanophényl)propionamide (composé 6).

F = 182°C, cristallisé avec 0,5 $H_2O$

. N-(2-naphtylsulfonyl) 3-[1-(pyrrolidinylcarbonyl) 2-(4-cyanophényl)éthylaminocarbonyl] tétrahydroisoquinoléine (composé 7)

F = 232°C, cristallisé avec 0,75 $H_2O$

. 1-(2-[(2-(2-naphtylsulfamoyl)2-indanyl)-carboxamido] 3-(4-cyanophényl) propionyl) pipéridine (composé 8)

F = 224°C, cristallisé avec $H_2O$

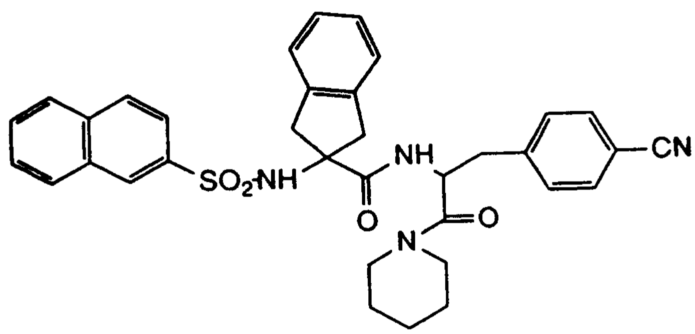

. 1-(2-[2-(8-quinolylsulfamoyl)-3-(4-méthoxyphényl)propionamido]-3-(4-cyanophényl)propionyl)pyrolidine (composé 9)

préparée à partir du sulfonamide racémique et du chlorhydrate de 1-[2-amino 3-(4-cyanophényl)]propionyl pyrrolidine, lévogyre

F=175° cristallisé avec 1,5 $H_2O$

. 1-(2-[2-(2-naphtyl sulfamoyl)-3-(4-benzyloxyphényl)propionamido]-3-(4-cyanophényl)propionyl)pyrrolidine (composé 10)

F=110°C, composé cristallisé avec 1$H_2O$

**2. Par condensation d'un alpha-amino acide avec un (4-cyanophényl)alanylamide suivie de la réaction avec le sulfochlorure.**

**2.1.** 1-(2-[2-(2-naphtylsulfamoyl)3-phényl propionamido] -3-(4-cyanophényl)propionyl)pyrrolidine (composé 4 et composé 4 bis).

a) A 0°C, on introduit dans 20 ml de $CH_3CN$, 1,75 g de N-(t-butoxycarbonyl) phénylalanine, 0,95 ml de N-éthyl morpholine, 3,34 g de BOP et 1,75 g de chlorhydrate de 1-(2-amino 3-(4-cyanophényl)propionyl)pyrrolidine puis 1, 6 ml de N-éthylmorpholine. Après 16 heures d'agitation à température ambiante, on élimine le solvant par distillation sous pression réduite et le résidu est dissous dans $CH_3COOC_2H_5$, en présence d'une solution saturée de $NaHCO_3$. La phase organique, lavée de la façon habituelle et séchée, est concentrée sous pression réduite et le résidu est purifié par chromatographie sur colonne de gel de silice en éluant avec $CH_2Cl_2/CH_3OH$ (98/2-V/V).

Le produit obtenu est dissous dans 50 ml de $CH_2Cl_2$ et, à 0°C, 50 ml de $CF_3COOH$ sont ajoutés. Lorsque le

milieu est revenu à température ambiante, on agite encore 30 minutes, puis on élimine les produits volatils par distillation sous pression réduite; après addition de 40 ml d'eau, le mélange est lyophilisé pour donner 2,9 g de trifluoroacétate.

b) On introduit lentement à 0°C, 1,75 ml de N-éthylmorpholine dans 35 ml d'une solution de 2,3 g du trifluoroacétate précédent dans $CH_2Cl_2$, puis 1,1 g de chlorure de 2-naphtalènesulfonyle en solution dans 10 ml de $CH_2Cl_2$. Après 4 heures sous agitation à température ambiante, la phase organique est lavée avec une solution aqueuse de HCl 0,1N puis à l'eau. Le résidu obtenu après distillation du solvant est chromatographié sur gel de silice en éluant avec un mélange de $CH_2Cl_2/CH_3OH$ (95/5-V/V), pour donner 1,95 g du composé 4 attendu.

Lorsque les deux produits de départ sont des énantiomères purs, on obtient dans ces conditions un seul des quatre stéréoisomères du composé 4.

Le produit (composé 4 bis) préparé à partir des deux énantiomères lévogyres précédemment décrits, cristallisé avec 0,25 $H_2O$, fond à 118°C.

$[\alpha]_D^{20}$ = - 19°5 (C = 1; DMSO)

On obtient par la même séquence réactionnelle:

. en partant de la N-(t-butoxycarbonyl)-L-phénylalanine et du chlorhydrate de la 1-[2-amino-3-(4-cyanophényl)propionyl]pyrrolidine, lévogyre, le composé 4 ter.
  $[\alpha]_D^{20}$ = + 15°5 (C = 1; DSMO)
. en partant de la N-(t-butoxy carbonyl)-O-méthyl-D-tyrosine et du chlorhydrate de la 1-[2-amino-3-(4-cyanophényl)propionyl]pyrrolidine, lévogyre, la 1-(2[2-(2-naphtyl sulfamoyle)-3-(4-méthoxyphényl)propionamido]3-(4-cyanophényl)propionyl)pyrrolidine hydratée (composé 3 bis)
  F = 143°C
  $[\alpha]_D^{20}$ = + 4°1 (C = 1; $CH_3OH$)
. en partant de la N-(t-butoxy carbonyl)-D-phénylalanine et du chlorhydrate de la 1-[2-amino-3-(4-cyanophényl)propionyl]pyrrolidine dextrogyre, la 1-(2-[2-(5-diméthylamino-1-naphtylsulfamoyl)-3-phénylpropionamido]-3-(4-cyanophényl)propionyl)pyrrolidine, (composé 11)
  F = 116°C
  $[\alpha]_D^{20}$ = - 4°5 (C = 1; DMSO)
. en partant des mélanges racémiques, la 1-(2-[2-(1-naphtylsulfamoyl)-3-(2-thiényl)propionamido]-3-(4-cyanophényl)propionyl)pyrrolidine (composé 12).
  F = 121°C

**2.2.** *1-(2-[(N-(2-naphtyl sulfamoyl)-5-méthoxy-2-indolyl) carboxamido]-3-(4-cyanophényl)propionyl)pyrrolidine* (composé 13).

a) A 0°C on introduit dans 100 ml de $CH_3CN$, 1,3 g d'acide 5-méthoxy-indole 2-carboxylique, 3,16 g de BOP et 2 g de chlorhydrate de 1-(2-amino-3-(4-cyanophényl)propionyl)pyrrolidine puis 2,5 ml de triéthylamine.

Après 16 heures d'agitation à température ambiante, on filtre, lave à l'éther et sèche la 1-(2-[(5-méthoxy-2-indolyl)carboxamido]-3-(4-cyanophényl)propionyl)pyrrolidine.

F = 204°C

b) A 0°C on ajoute 0,23 g d'une suspension d'hydrure de sodium à 60% dans l'huile à une suspension de 2,2 g du produit précédent dans 100 ml de THF.

Après 1 heure d'agitation à 5°C, on ajoute vers 0° 1,3 g de chlorure de 2-naphtalène sulfonyle en solution dans 20 ml de THF et agite à température ambiante durant 16 heures puis vers 50°C durant 3 heures. On filtre et évapore le filtrat sous pression réduite. Le résidu est chromatographié sur une colonne de silice en éluant avec $CH_2Cl_2$/cyclohexane (70/30 - v/v) pour donner le produit attendu hémihydraté qui fond à 186°C.

De la même manière, en partant de l'acide indole-2 carboxylique, on isole le 1-(2-[(N(2-naphtyl sulfamoyl)-2-indolyl)carboxamido]-3-(4-cyanophényl)propionyl)pyrrolidine (composé 14)

F = 180°C

**3. Par substitution d'un sulfonamide de formule II pour obtenir un composé dans lequel $R_1$est différent de H.**

. 1-(2-[2-(N-méthyl 2-naphtylsulfamoyl 3-phényl propionamido] 3-(4-cyanophényl)propionyl) pyrrolidine (composé 15)

On dissout 2 g de composé 4 dans 20 ml de diméthylformamide à 0°C et on ajoute 0,475 g de $K_2CO_3$ et 0,214 ml de $ICH_3$. Après 24 heures à 5°C, on ajoute 20 ml de $H_2O$ et 40 ml de $CH_2Cl_2$. La phase organique décantée est lavée, séchée et amenée à sec. Le résidu est recristallisé dans $(CH_3)_2CHOH$ pour donner 1,76 g de produit final fondant à 186°C.

L'énantiomère préparé à partir du composé 4 bis décrit en C-2 est dextrogyre.

$[\alpha]^{20}_{D} = 40°$ (C = 1; $CH_3OH$)

**4. Par réaction d'un sulfonamide et d'un ester de la 4-cyanophénylalanine suivie d'une saponification et d'une acylation.**

. 1-(2-[2-(4-méthylphényl sulfamoyl)-3-(4-chlorophényl)propionamido]-3-(4-cyanophényl)propionyl)hexahydroazépine (composé 16).

a) On fait réagir dans les conditions décrites au § (C.1) 4 g de N-(4-tolyl sulfonyl)-4-chlorophényl)alanine et 4,1 g de chlorhydrate de l'ester éthylique de la 4-cyanophénylalanine et obtient par les traitements habituels 3,7 g de 2-(2-(4-tolylsulfamoyl)-3-(4-chlorophényl)-propionamido)3-(4-cyanophényl)propionate d'éthyle qui fond à 82°C.

Cet ester est hydrolysé par une solution de 1 g de KOH dans un mélange de 10 ml d'eau et 20 ml d'éthanol pour donner après acidification 2,5 g de l'acide correspondant qui fond à 104°C.

b) A 0°C on ajoute à une solution de 2,5 g de l'acide précédent dans 80 ml de $CH_3CN$ 2 g de B.O.P., 1,6 ml de N-éthylmorpholine puis 0,5 g d'hexaméthylenimine. Après 16 heures d'agitation vers 20°C, on évapore le solvant. Le résidu est dissout dans $CH_2Cl_2$. La phase organique est lavée de la façon habituelle séchée puis concentrée sous pression réduite.

Après chromatographie sur gel de silice en éluant avec un mélange $CH_2Cl_2/CH_3OH$ (v/v : 95/5), on obtient 1,3 g du produit attendu.

F = 194°C

De la même manière on prépare la (2-[2-(1-naphtylsulfamoyl)-3-(2,4-diméthylphényl)propionamido] 3-(4-cyanophényl)propionyl)diméthylamine hémi-hydraté (composé 17).

F = 140°C

De la même manière on prépare la 1-(2-[2-(5-isoquinolylsulfamoyl)-2-méthyl-3-phényl-propionamido]-3-(4-cyanophényl)-propionyl)-pyrrolidine (composé 18).

F = 264°C

**D. <u>Préparation d'imidoesters intermédiaires dans la préparation des amidines à partir des nitriles</u>**

**1. à partir du composé 5 et de $C_2H_5OH$.**

On introduit rapidement 2 g du composé 5 dans 20 ml de $C_2H_5OH$ anhydre saturé à 0°C de HCl. Après une nuit d'agitation à une température comprise entre 0°C et 5°C, on élimine le solvant par distillation à une température inférieure à 25°C, pour obtenir le chlorhydrate du produit cherché.

**2. A partir du composé 4 et de $CH_3OH$.**

On introduit rapidement 5 g du composé 4 dans 100 ml de $CH_3OH$ anhydre saturé à 0°C de HCl. Après une nuit d'agitation vers 0°C, on élimine le solvant par distillation à une température inférieure à 22°C, pour isoler le chlorhydrate de l'imidoester.

Pour obtenir la base correspondante, le chlorhydrate est dissous dans 100 ml de $CH_2Cl_2$ puis on ajoute vers 5°C de la triéthylamine jusqu'à obtenir un pH de 7,5 (mesuré en milieu aqueux). La phase organique est alors lavée 5

fois avec 30 ml d'eau vers 20°C, séchée et concentrée pour donner 5,2 g d'imidoester.

Les formules développées et les caractéristiques physico-chimiques des produits préparés comme décrit dans les exemples qui suivent figurent dans le tableau I ; A/B représente les proportions relatives des 2 racémiques.

## Exemple 1

2 g du chlorhydrate d'imidoester du composé 5, préparé selon D-1, sont dissous dans 20 ml d'isopropanol anhydre et 1,6 ml de n-propylamine sont introduits dans la solution. Après 2 heures d'agitation, le solvant est éliminé et le résidu chromatographié sur gel de silice en éluant avec un mélange $CH_2Cl_2/CH_3OH$ (9/1-V/V). On obtient ainsi 1,7 g du chlorhydrate du produit attendu.

## Exemple 2

1 g du chlorhydrate d'imidoester du composé 5, préparé selon D-1, sont dissous dans 10 ml de $C_2H_5OH$ anhydre. On ajoute 0,14 ml de 1,2-éthanediamine et on maintient le milieu vers 70°C durant 1 heure 30. Le solvant est éliminé sous pression réduite et le résidu purifié par chromatographie sur gel de silice en éluant avec un mélange $CH_2Cl_2/CH_3OH$ (92/8-V/V).

## Exemple 3

A une solution de 2 g de chlorhydrate d'imidoester du composé 4 préparé selon D-1, dans 60 ml de $C_2H_5OH$ anhydre, on ajoute à 5°C, 2,1 ml de triéthylamine et 0,33 ml de 1-aminopentanol. Après 16 heures d'agitation à température ambiante, on évapore le solvant sous pression réduite et on chromatographie le résidu sur gel de silice en éluant avec un mélange $CH_2Cl_2/CH_3OH$ (9/1-V/V) pour obtenir 1,5 g de chlorhydrate.

## Exemple 4

On introduit à 5°C dans une solution de 4,5 g de chlorhydrate d'imidoester du composé 4, 3,5 ml de triéthylamine et 2,2 g de N-(t-butyloxy-carbonyl)propane diamine-1,3. Après 20 heures à température ambiante le solvant est éliminé par distillation sous pression réduite et le résidu est dissous dans 50 ml de $CH_2Cl_2$. La solution organique est lavée avec une solution aqueuse de NaOH 0,1N, puis à l'eau et séchée. Le résidu obtenu après concentration, cristallise dans un mélange de $[(CH_3)_2CH]_2O$ et $CH_3COOC_2H_5$ pour donner 2,5 g du produit recherché.

## Exemple 5

A 5°C, on introduit dans une solution de 4 g du produit obtenu dans l'exemple 4 dans 40 ml de $CH_3COOC_2H_5$ , 40 ml de $CH_3COOC_2H_5$ saturé de HCl. Après 2 heures vers 20°C, le précipité est isolé, lavé avec $CH_3COOC_2H_5$ puis dissous dans un mélange de 40 ml de $CH_2Cl_2$ et 20 ml de solution aqueuse de NaOH 0,5 N. Après 16 heures d'agitation vers 20°C, on sépare la phase organique et on la traite de façon habituelle pour obtenir 2,1g du produit attendu après recristallisation dans un mélange de $CH_3COOC_2H_5$ et $CH_3OH$.

## Exemple 6

On introduit 2 g de l'imidoester du composé 4 bis préparé selon D-2 dans 30 ml de $CH_3OH$ anhydre et on ajoute 0,82 g de trans 4-[N-(t-butoxycarbonyl)aminométhyl]cyclohexylméthylamine et puis quelques gouttes de $CH_3OH$ saturé en HCl jusqu'à obtenir un pH 8 (mesuré dans l'eau).

Après 52 heures à température ambiante, on évapore $CH_3OH$, on ajoute 30 ml de $CH_2Cl_2$ dans le milieu, puis l'élimine sous pression réduite ; on introduit à 5°C le résidu obtenu dans 30 ml de $CH_3COOC_2H_5$ saturé à 15°C de HCl. Après retour à la température ambiante, on laisse 1/2 heure sous agitation puis on élimine le solvant avant de purifier le résidu par chromatographie sur gel de silice en éluant avec un mélange de $CH_2Cl_2/CH_3OH$ (80/20-V/V).

Après recristallisation dans le 1-propanol, on isole 1,8 g du produit mentionné dans le tableau I.

## Exemple 7

a) (N,N-diméthyl)4-[aminométhyl] cyclohexylméthylamine (trans)

On introduit à 0°C dans 50 ml de dioxanne contenant 5 g d'acide trans 4-aminométhylcyclohexylcarboxylique,

63,6 ml d'une solution aqueuse de NaOH N et 1,28 g de MgO puis lentement 6,94 g de di(t-butyl)carbonate en solution dans 20 ml de dioxanne. Après 20 heures à température ambiante, on filtre, on élimine le solvant, et on reprend le résidu dans 100 ml de $H_2O$ et lave la phase aqueuse par $(C_2H_5)_2O$ avant de l'acidifier jusqu'à pH 2 par addition de $KHSO_4$; on extrait ensuite dans $CH_3COOC_2H_5$ pour obtenir 7,3 g d'acide N-(t-butoxycarbonyl) 4-aminométhyl cyclohexyl carboxylique, trans qui fond à 125°C.

Ce composé est ensuite dissous dans un mélange de 20 ml de $CH_2Cl_2$ et 25 ml de $(CH_3)_2NCHO$, dans lequel on introduit 4,8 g d'hydroxybenzotriazole puis 6,15 g de N,N'-dicyclohexylcarbodiimide en solution dans 50 ml de $CH_2Cl_2$. Après 2 heures d'agitation, on ajoute 4g de $(CH_3)_2NH$ anhydre et laisse sous agitation durant 16 heures. Le précipité est alors séparé, la phase organique est lavée plusieurs fois par une solution aqueuse saturée de $NaHCO_3$ puis à l'eau. Après séchage, concentration et chromatographie du résidu sur gel de silice en éluant avec $CH_3COOC_2H_5$, on isole 5,8 g de N,N-diméthyl-N'-(t-butoxycarbonyl)4-aminométhylcyclohexyl carboxamide, trans qui fond à 94°C.

Ce composé est dissous dans 50 ml de $CH_3COOC_2H_5$ saturé de HCl et après une heure, on filtre le précipité de chlorhydrate apparu, qui, traité par une base donne 3,5 g de N,N-diméthyl 4-aminométhylcyclohexylcarboxamide, trans, sous forme d'huile.

Cette huile est dissoute dans 10 ml de tétrahydrofuranne dans lequel on introduit ensuite à 0°C, 25 ml de solution $LiAlH_4$, 1M dans le même solvant. Après 16 heures d'agitation à température ambiante, on refroidit à 0°C et on ajoute 0,9 ml d'eau glacée puis 2,7 ml d'une solution aqueuse de NaOH à 15% (p/v) et enfin 0,9 ml d'eau. Le précipité est éliminé et le solvant est évaporé par distillation sous pression réduite pour donner la diamine attendue qui distille à 60°C sous 1 Pa.

b) En faisant réagir selon le mode opératoire décrit à l'exemple 3, la (N,N-diméthyl)4-aminométhylcyclohexylméthylamine, trans et l'imidoester du composé 4 bis, on obtient après recristallisation dans l'isopropanol l'énantiomère pur décrit dans le tableau I.

**Préparation du composé de l'exemple 46 à partir de celui de l'exemple 38**

Sous atmosphère inerte, on ajoute à une solution de 0,5 g de composé 38 dans 10 ml de méthanol anhydre, 0,08 g d'acide aminoiminométhanesulfonique et 0,1 ml de triéthylamine. Après 16 heures à environ 20°C, on évapore le solvant et reprend le résidu dans 20 ml de solution aqueuse de NaOH 1N à température voisine de 0°C et on extrait au dichlorométhane. La phase organique est séchée, concentrée et le résidu est chromatographié sur une colonne de silice (éluant dichlorométhane/méthanol 9/1-V/V) puis avec un mélange méthanol/solution aqueuse de $NH_4$ OH concentré (7/3 - V/V). Après recristallisation dans un mélange d'éthanol/acétate d'éthyle 8/2 V/V), on isole le produit attendu sous forme de base dont on prépare le dichlorhydrate par action de HCl dans l'éthanol.

F = 185°C (2 HCl, $H_2O$).

Selon le mode opératoire général décrit dans l'exemple 3, on prépare avec les amines adéquates le composé de l'exemple 20 à partir du composé 4 bis, le composé de l'exemple 60 à partir du composé 4 ter, le composé de l'exemple 58 à partir du composé 3 bis, le composé de l'exemple 64 à partir du composé 10.

Selon le mode opératoire décrit dans l'exemple 6, on prépare avec les amines adéquates les composés des exemples 45, 47 et 48 à partir du composé 4 bis.

**Exemple 61**

a) N-(butyloxycarbonyl)-4-(aminométhyl)cyclohexylméthylamine (cis)

A 0°C on ajoute par portions 1,6 g de terbutylate de potassium à une solution de 2 g de dichlorhydrate de 1,4-[diméthylamino]-cyclohexane, cis (obtenue selon la méthode décrite dans Ber. 71 B, 759 (1938)) dans 70 ml de méthanol anhydre puis, une solution de 2,1 g de dicarbonate de diter-butyle dans 100 ml de méthanol.

On chauffe 16 heures vers 35°C, filtre le précipité et évapore le solvant sous pression réduite. Le produit attendu est isolé par chromatographie sur une colonne de silice en éluant au $CH_2Cl_2$/MeOH : 95/5 puis 80/20 (v/v)

F = 201°C

b) En faisant réagir l'amine précédente avec 2 g de l'imido ester du composé 4 bis préparé selon D-2 dans les conditions décrites à l'exemple 6, on obtient le produit attendu qui est dissout dans 15 ml d'HCl, filtré puis extrait de la phase aqueuse par 3 fois 7 ml de butanol. On évapore le solvant sous pression réduite. Le résidu est repris dans l'eau et lyophylisé pour donner l'énantiomère pur décrit dans le tableau I.

**Exemple 67**

a) 4( N,N-diméthylaminométhyl)cyclohexylméthylamine, cis

- *cyclohexyl-1,4-diméthyanol, cis*

A 0°C on ajoute lentement 328 ml d'une solution 1M dans le THF d'aluminohydrure de lithium à une solution de 66g de cylohexyl-1,4-dicarboxylate d'éthyle, cis dans 500 ml de THF.

On agite 16 heures à température ambiante et ajoute vers 0°C 13 ml d'eau, 39 ml d'une solution aqueuse de NaOH à 15% (pds/pds), puis à nouveau 13 ml d'eau. On filtre les sels, évapore le solvant sous pression réduite, distille le résidu à 120-124° sous 45 x $10^{-6}$ bar (4.5 Pa). On obtient 37 g du produit attendu.

- *cyclohexyl-1,4-di-paratoluène sulfonate (cis)*

A 0°C on ajoute à une solution de 41 g de chlorure de paratoluène sulfonyle et 28 ml de triéthylamine dans 35 ml de THF une solution de 10g de l'alcool précédent dans 70 ml de THF.

On agite vers 25° durant 24 heures et chauffe vers 50°C durant 3 heures.

Après refroidissement, on ajoute alors 50 ml d'une solution saturée de NaCl et 50 ml d'une solution d'HCl environ 1N. On évapore le solvant sous pression réduite et reprend le résidu par 300 ml d'éther et 200 ml de NaOH 2N et agite 16 heures à température ambiante.

On décante et extrait la phase aqueuse au dichlorométhane. Les phases organiques sont séchées et on distille le solvant sous pression réduite. On isole 29 g du produit attentu. F = 91°C.

- *4-(N,N-diméthylaminométhyl)cyclohexylméthylamine, cis*

On agite à 25°C en autoclave durant 48 heures 14 g du ditosylate précédent dans une solution méthanolique saturée d'ammoniac. Après évaporation sous pression réduite, on reprend le résidu par du $CH_2Cl_2$ et HCl 1N, décante, basifie la phase aqueuse par NaOH 2N et, extrait au $CH_2Cl_2$. Après évaporation du solvant sous pression réduite on chromatographie le résidu sur silice en éluant avec un mélange $CH_2Cl_2/CH_3OH$:70/30 (v/v) et isole 6g de 4-aminométhylcyclohexyl paratoluène sulfonate, cis.

On ajoute le tosylate précédent à une solution saturée de diméthylamine dans le méthanol et chauffe en autoclave à 70°C durant 20 heures.

Après évaporation sous pression réduite, on reprend le résidu par du $CH_2Cl_2$, 2 ml d'eau et 2g de KOH solide.

La phase organique est séchée et concentrée sous vide. On isole, après chlorhydratation et recristallisation dans l'éthanol 3g de dichlorhydrate du produit attendu qui fond à 252°C.

b) Composé 67

On fait réagir 0,9 g du dichlorhydrate précédent avec 2,4 g du chlorhydrate d'imidoester du composé 4 bis et 1,2 ml de N-éthylmorpholine dans 100 ml de méthanol. Après 16 heures d'agitation à 40°C, on évapore le solvant et traite selon le mode opératoire décrit dans l'exemple 61, paragraphe b pour obtenir l'énantiomère pur décrit dans le tableau 1.

**Exemple 69**

(Cas où $R_2$ et $R'_2$ forment une double liaison) 1(2-[N-(2-naphtylsulfamoyl)-2-indolyl carboxamido] 3-(4-(N-[4-(diméthylaminométhyl)trans-cyclohexylméthyl]amidino)phényl)propionyl) pyrrolidine, dichlorhydrate, $4H_2O$.

Ce composé est préparé à partir du composé 13 selon le mode opératoire général décrit à l'exemple 1.

F = 230°C

**Exemple 70**

1-(2-[(N-(2-naphtylsulfamoyl)-5-méthoxy-2-indolyl) -carboxamido]-3-(4-(N-[4-(diméthylaminométhyl)trans-cyclohexylméthyl]amidino)phényl)propionyl) pyrrolidine.

Ce composé est préparé de la même façon à partir du composé 12.

F = 230°C.

**TABLEAU 1**

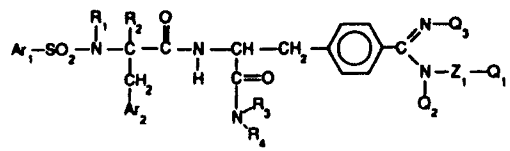

| ex. | $Ar_1$ | $R_1$ | $Ar_2$ | $R_2$ | $NR_3R_4$ | $Z_1$ | $Q_1$ | $NQ_2$ | $NQ_3$ | sel (solvate) | F °C. | A/B | $[\alpha]_D^{20}$ (1) |
|---|---|---|---|---|---|---|---|---|---|---|---|---|---|
| 1 | 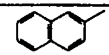 | H | 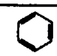 | H | 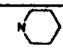 | $(CH_2)_2$ | $CH_3$ | NH | NH | HCl, 2,5 $H_2O$ | 210 | 40/60 | |
| 2 | 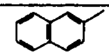 | H | 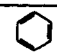 | H | 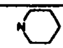 | rien | H | $-N-(CH_2)_2N-$ | | HCl, 2,5 $H_2O$ | 228 | 45/55 | |
| 3 | 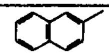 | H | 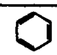 | H | 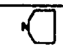 | $(CH_2)_5$ | OH | NH | NH | HCl, $H_2O$ | 231 | 60/40 | |
| 4 | 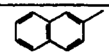 | H | 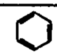 | H | 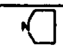 | $(CH_2)_3$ | $NHCOOC(CH_3)_3$ | NH | NH | Base, 0,5 $H_2O$ | 150 | 50/50 | |
| 5 | 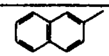 | H | 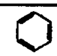 | H | 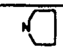 | rien | H | $-N-(CH_2)_3-N-$ | | Base, 0,5 $H_2O$ | 171 | 25/75 | |
| 6 | 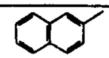 | H | 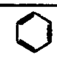 | H | 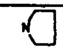 | 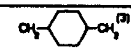 (3) | $NH_2$ | NH | NH | 2HCl, 4 $H_2O$, 0,5 $C_3H_2O$ | 260 | 0/100 (2) | +19°2 |
| 7 | 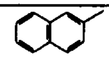 | H | 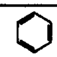 | H | 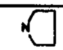 | 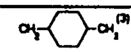 (3) | $N(CH_3)_2$ | NH | NH | 2HCl, 3 $H_2O$ | 225 | 0/100 (2) | +21°4 |
| 8 | 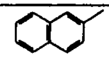 | H | 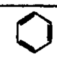 | H | 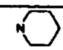 | $-CH(CH_3)-$ | $CH_3$ | NH | NH | Base, $H_2O$ | 192 | 40/50 | |
| 9 | 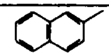 | H | 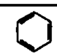 | H | 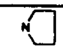 | $(CH_2)_2$ | $CH_3$ | NH | NH | HCl, $H_2O$ | 262 | 30/60 | |

| | | | | | | | | | | | | | |
|---|---|---|---|---|---|---|---|---|---|---|---|---|---|
| 10 | 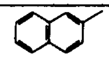 | H | 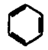 | H | 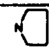 | $(CH_2)_3$ | $CH_3$ | NH | NH | HCl, 1/3 $H_2O$ | 270 | 50/50 | |
| 11 | 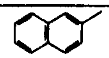 | H | 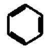 | H | | $(CH_2)_3$ | $CH_3$ | $NCH_3$ | NH | Base, 1,5 $H_2O$ | 200 | 60/40 | |
| 12 | 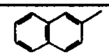 | H | 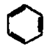 | H | | $(CH_2)_3$ | $N(CH_3)_2$ | NH | NH | 2HCl, 0,5 $H_2O$ | 172 | 50/50 | |
| 13 | 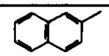 | 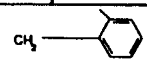 | | H | | $(CH_2)_3$ | $N(CH_3)_2$ | NH | NH | HCl, 2 $H_2O$ | 184 | 50/50 | |
| 14 | 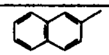 | H | 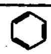 | H | | $(CH_2)_4$ | $N(CH_3)_2$ | NH | NH | HCl, 1,5 $H_2O$ | 265 | 50/50 | |
| 15 | 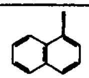 | H | 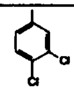 | H | 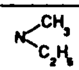 | 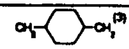 | $N(CH_3)_2$ | NH | NH | 2HCl, 6 $H_2O$ | 260 | 50/50 | |
| 16 | 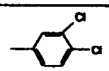 | H | 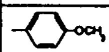 | H | 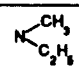 | 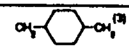 | $N(CH_3)_2$ | NH | NH | 2HCl, 3 $H_2O$ | 230 | 50/50 | |
| 17 | 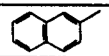 | H | 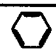 | H | | 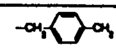 | $NH_2$ | NH | NH | 2HCl, 4 $H_2O$ | 245 | 35/65 | |
| 18 | 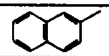 | H | 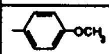 | H | | 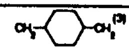 | $N(CH_3)_2$ | NH | NH | 2HCl, 2 $H_2O$ | 222 | 50/50 | |
| 19 | 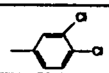 | H | 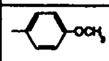 | H | | 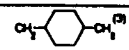 | $N(CH_3)_2$ | NH | NH | 2HCl, 3,5 $H_2O$ | 244 | 50/50 | |
| 20 | 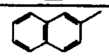 | H | 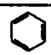 | H | | 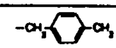 | $N(CH_3)_2$ | NH | NH | 2HCl, 3 $H_2O$ | 255 | 0/100(2) | +24°5 |
| 21 | 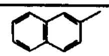 | H | 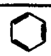 | H | | $(CH_2)_3$ | 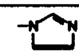 | NH | NH | $2CF_3COOH$, 2 $H_2O$ | 190 | 35/65 | |
| 22 | 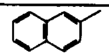 | H | 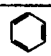 | H | | $(CH_2)_2$ | 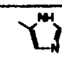 | NH | NH | 2HCl, 2 $H_2O$ | 220 | 65/35 | |
| 23 | 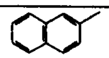 | H | 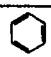 | H | | $(CH_2)_3$ | 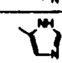 | NH | NH | 2HCl, 2 $H_2O$ | 242 | 50/50 | |

| | | | | | | | | | | | | | |
|---|---|---|---|---|---|---|---|---|---|---|---|---|---|
| 24 | 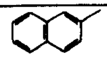 | H | 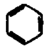 | H | | $(CH_2)_5$ | 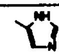 | NH | NH | 3HCl, 3 $H_2O$ | 240 | 50/50 | |
| 25 | 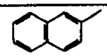 | $CH_3$ | 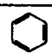 | H | | $(CH_2)_3$ | 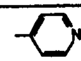 | NH | NH | 2HCl, 4 $H_2O$ | 274 | 80/20 | |
| 26 | 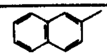 | $CH_3$ | 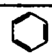 | H | | $(CH_2)_3$ | 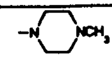 | NH | NH | 2HCl, 2 $H_2O$ $C_3H_7OH$ | 245 | 40/60 | |
| 27 | 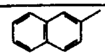 | H | 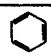 | H | | $(CH_2)_2$ | 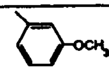 | NH | NH | Base, $H_2O$ | 150 | 15/85 | |
| 28 | 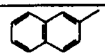 | H | 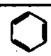 | H | | $(CH_2)_3$ | $-COOC_2H_5$ | NH | NH | HCl, $H_2O$ | 228 | 50/50 | |
| 29 | 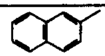 | H | 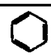 | H | | $(CH_2)_5$ | $-CONH_2$ | NH | NH | HCl, 2 $H_2O$ | 256 | 65/35 | |
| 30 | 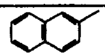 | H | 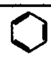 | H | | $(CH_2)_6$ | -OH | NH | NH | HCl, 4 $H_2O$ | 160 | 75/25 | |
| 31 | 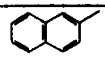 | H | 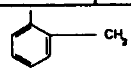 | | | 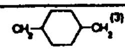 | $N(CH_3)_2$ | NH | NH | 2HCl, 4 $H_2O$ | 275 | 50/50 | |
| 32 | 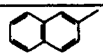 | H | 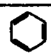 | H | | $(CH_2)_4$ | $NHCOOC(CH_3)_3$ | NH | NH | Base | 165 | 50/50 | |
| 33 | 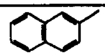 | H | 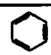 | H | | $(CH_2)_5$ | $NHCOOC(CH_3)_3$ | NH | NH | Base, 0,5 $H_2O$ | 180 | 55/45 | |
| 34 | 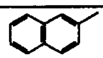 | H | 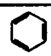 | H | | 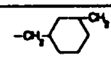 | $NHCOOC(CH_3)_3$ | NH | NH | HCl, 2 $H_2O$ | 200 | 40/60 | |
| 35 | 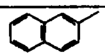 | H | 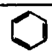 | H | | $(CH_2)_6$ | $NHCOOC(CH_3)_3$ | NH | NH | HCl, $H_2O$ | 204 | 50/50 | |
| 36 | 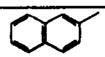 | H | 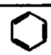 | H | | $(CH_2)_7$ | $NHCOOC(CH_3)_3$ | NH | NH | HCl, $H_2O$ | 205 | 50/50 | |
| 37 | 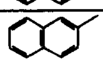 | H | 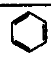 | H | | $(CH_2)_8$ | $NHCOOC(CH_3)_3$ | NH | NH | HCl, 1,5 $H_2O$ | 215 | 50/50 | |
| 38 | 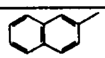 | H | 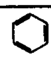 | H | | $(CH_2)_4$ | $NH_2$ | NH | NH | 2HCl, 1,5 $H_2O$ | 230 | 50/50 | |

| | | | | | | | | | | | | | |
|---|---|---|---|---|---|---|---|---|---|---|---|---|---|
| 39 | 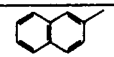 | H | 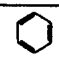 | H | 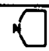 | $(CH_2)_5$ | $NH_2$ | NH | NH | 2HCl, 2,5 $H_2O$ | 274 | 50/50 | |
| 40 | 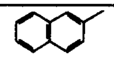 | H | 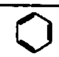 | H | 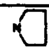 | $(CH)_6$ | $NH_2$ | NH | NH | Base, 2/3 $H_2O$ | 170 | 50/50 | |
| 41 | 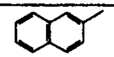 | H | 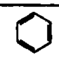 | H | 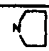 | $(CH_2)_7$ | $NH_2$ | NH | NH | 2HCl, 4 $H_2O$ | 192 | 55/45 | |
| 42 | 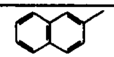 | H | 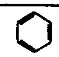 | H | 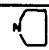 | $(CH_2)_8$ | $NH_2$ | NH | NH | 2HCl, 2 $H_2O$ | 228 | 60/40 | |
| 43 | 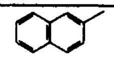 | H | 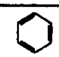 | H | 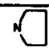 | $(CH)_9$ | $NH_2$ | NH | NH | $2CF_3COOH$, 2 $H_2O$ | 238 | 50/50 | |
| 44 | 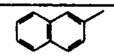 | H | 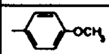 | H | 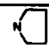 | $(CH_2)_9$ | $NH_2$ | NH | NH | 2HCl, 2 $H_2O$ | 215 | 50/50 | |
| 45 | 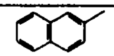 | H | 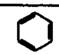 | H | 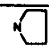 | $(CH_2)_7$ | $NH_2$ | NH | NH | 2 HCl, 2,5 $H_2O$, 0,5 $C_3H_7OH$ | 245 | 0/100(2) | +38°6 |
| 46 | 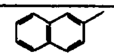 | H | 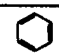 | H | 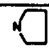 | $(CH_2)_4$ | 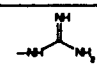 | NH | NH | 2HCl, 2 $H_2O$ | 185 | 50/50 | |
| 47 | 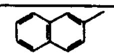 | H | 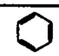 | H | | 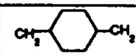 | $NH_2$ | NH | NH | $2(COOH)_2$ 0,75 $H_2O$ | 207 | 0/100(2) | +26°6 |
| 48 | 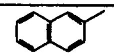 | H | 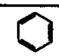 | H | | 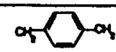 | $NH_2$ | NH | NH | 2HCl, 2,5 $H_2O$ | 275 | 0/100(2) | +23° |
| 49 | 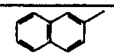 | H | 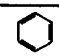 | H | | 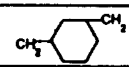 | $NH_2$ | NH | NH | 2HCl, 3,5 $H_2O$ | 222 | 50/50 | |
| 50 | 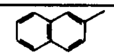 | H | 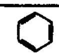 | H | | 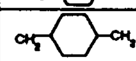 | $NH_2$ | NH | NH | $2(COOH)_2$ 0,25 $H_2O$ | 120 | 50/50 | |
| 51 | 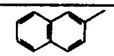 | H | 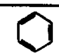 | H | | $(CH_2)_5$ | 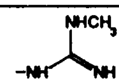 | NH | NH | 2HCl, 4 $H_2O$ | 210 | 50/50 | |

| | | | | | | | | | | | | | |
|---|---|---|---|---|---|---|---|---|---|---|---|---|---|
| 52 | 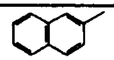 | H | 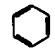 | H | 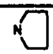 | $(CH)_5$ | 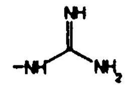 | NH | NH | $3CF_3COOH$, $5H_2O$ | 245 | 50/50 | |
| 53 | 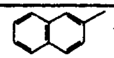 | H | 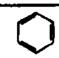 | H | 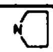 | $(CH_2)_6$ | 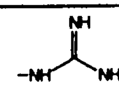 | NH | NH | 3CF3COOH, 1,5 $H_2O$ | 218 | 50/50 | |
| 54 | 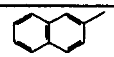 | H | 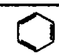 | H | 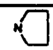 | $(CH_2)_7$ | 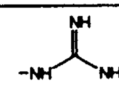 | NH | NH | 2HCl, 2 $H_2O$ | 240 | 55/45 | |
| 55 | 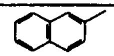 | H | 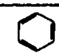 | H | 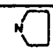 | $(CH_2)_8$ | 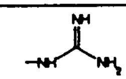 | NH | NH | $2CF_3COOH$, 0,5 $H_2O$ | 205 | 55/45 | |
| 56 | 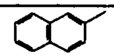 | $CH_3$ | 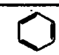 | H | 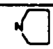 | $(CH_2)_3$ | $NCH_3$-$(CH_2)_3NH_2$ | NH | NH | 3HCl, 5 $H_2O$ | 246 | 70/30 | |
| 57 | 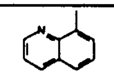 | H | 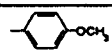 | H | 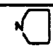 | 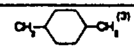 | $N(CH_3)_2$ | NH | NH | 3 HCl, 4 $H_2O$ | 260 | 50/50 | |
| 58 | 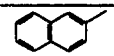 | H | 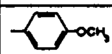 | H | 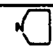 | 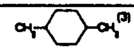 | $N(CH_3)_2$ | NH | NH | 2 HCl, 3 $H_2O$ | 245 | 0/100(2) | +16°1 |
| 59 | 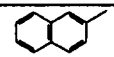 | H | 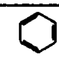 | H | 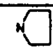 | $(CH_2)_2$ | 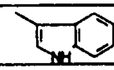 | NH | NH | HCl | 228 | 50/50 | |
| 60 | 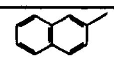 | H | 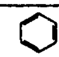 | H | | 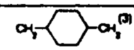 | $N(CH_3)_2$ | NH | NH | 2 HCl, 3 $H_2O$ | 220 | 100(4)/0 | -19°7 |
| 61 | 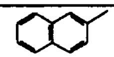 | H | 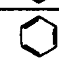 | H | | 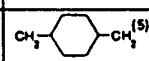 | $NH_2$ | NH | NH | 2 HCl, 2,5 $H_2O$ | 222 | 0/100(2) | +26° |
| 62 | 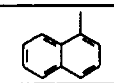 | H | 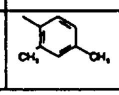 | H | | 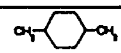 | $N(CH_3)_2$ | NH | NH | | 208 | | |

| | | | | | | | | | | | | | |
|---|---|---|---|---|---|---|---|---|---|---|---|---|---|
| 63 | 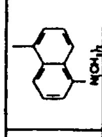 | H | 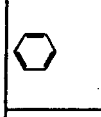 | H | 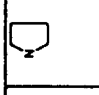 | 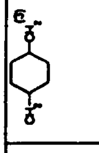 | N(CH3)2 | NH | NH | 3HCl, 3H2O | 185 | 100(4)/0 | +50°9 |
| 64 | 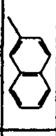 | H | 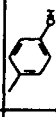 | H | 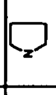 | 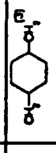 | N(CH3)2 | NH | NH | 2CF3COOH, 3H2O | 186 | 50/50 | |
| 65 | 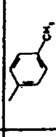 | H | 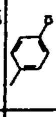 | H | 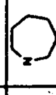 | 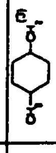 | N(CH3)2 | NH | NH | 2H2O | | 40/60 | |
| 66 | 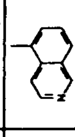 | H | 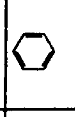 | CH3 | 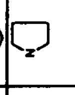 | 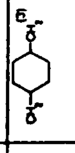 | N(CH3)2 | NH | NH | 3HCl, 3H2O | | 60/40 | |
| 67 | 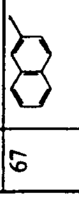 | H | 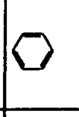 | H | 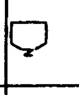 | 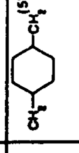 | N(CH3)2 | NH | NH | 2HCl, 4H2O | | 0/100(2) | +52° |
| 68 | 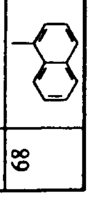 | H | 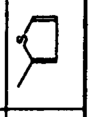 | H | 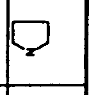 | 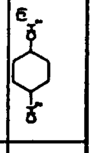 | N(CH3)2 | NH | NH | 2HCl, 3H2O | | 50/50 | |

(1) c = 1, $CH_3OH$
(2) 1 seul énantiomère dans B
(3) Cyclohexyl forme trans
(4) un seul énantiomère dans A
(5) Cyclohexyl forme cis

## Revendications

1. Composés de formule I

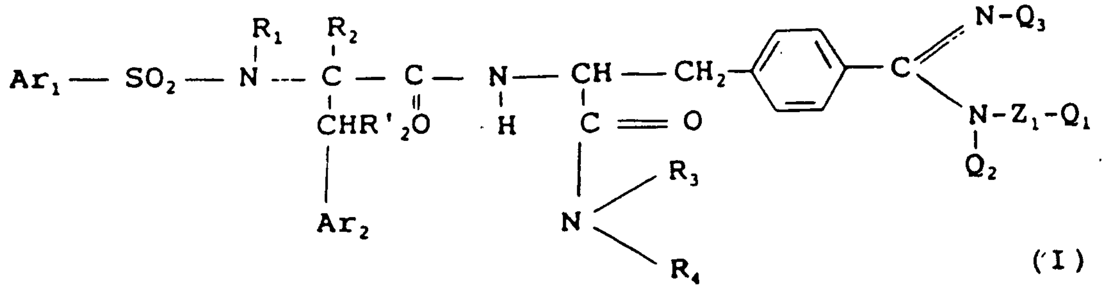

(I)

dans laquelle

$Ar_1$ représente naphtyle, phényle, quinolyle ou isoquinolyle éventuellement substitués par Cl, F, $(C_1$-$C_4)$alkyle, $(C_1$-$C_4)$alkoxy, hydroxyle ou $(C_1$-$C_4)$dialkylamino,
$Ar_2$ représente phényle ou thiényle, éventuellement substitués par Cl, F, $(C_1$-$C_4)$alkyle, $(C_1$-$C_4)$alkoxy ou hydroxyle;
$R_1$, $R_2$ et $R'_2$ représentent indépendamment l'un de l'autre H, $(C_1$-$C_4)$alkyle ou $R_1$ ne représente rien et N est lié à $Ar_2$ et, éventuellement $R_2$ et $R'_2$ forment une double liaison,
ou $R_1$ ou $R_2$ est lié à $Ar_2$ et représente $(C_1$-$C_3)$ alkylène;
$R_3$ et $R_4$, identiques ou différents, représentent H, $(C_1$-$C_4)$alkyle ou forment avec l'atome d'azote auquel ils sont liés un hétérocycle saturé en $(C_5$-$C_7)$ choisi parmi pyrrolidine, pipéridine et hexahydroazépine;
$Z_1$ représente $(C_1$-$C_{12})$alkylène interrompu ou prolongé éventuellement par $(C_5$-$C_7)$cycloalkyle ou phényle;
$Q_1$ représente méthyle, amino, $(C_1$-$C_4)$alkoxycarbonylamino, $(C_1$-$C_4)$alkylamino, di$(C_1$-$C_4)$alkylamino, pyrrolidinyle, pipéridino, morpholino, pipérazinyle, $(C_1$-$C_4)$alkyl-4 pipérazinyle, amidino, $(C_1$-$C_4)$alkylamidino, guanidino, $(C_1$-$C_4)$alkylguanidino, pyridyle, imidazolyle, pyrimidinyle, indolyle, hydroxy, $(C_1$-$C_4)$alkoxy, $(C_2$-$C_8)$alkoxycarbonyle, amino$(C_1$-$C_4)$alkyl-N-$(C_1$-$C_4)$alkylamino, carbamoyle ou phényle éventuellement substitué par Cl, F, ($C_1$ à $C_4$)alkyle, ($C_1$ à $C_4$)alkoxy ou hydroxyle;
$Q_2$ représente H ou $(C_1$-$C_4)$alkyle;
$Q_3$ représente H ou $(C_1$-$C_4)$alkyle ou $Q_1$ et $Q_3$ sont liés pour former un hétérocycle et représentent ensemble $(C_2$-$C_3)$alkylène tandis que $Z_1$ ne représente rien, sous forme d'énantiomères purs ou de leurs mélanges en proportions quelconques,
ainsi que leurs sels avec des acides.

2. Composés selon la revendication 1 de formule I dans laquelle $NR_3R_4$ représente pyrrolidinyle.

3. Composés selon l'une des revendications 1 et 2 de formule I dans laquelle $Z_1$ représente alkylène en $C_4$ à $C_9$ et $Q_1$ comporte un atome d'azote fixé à $Z_1$.

4. Composés selon la revendication 3, dans lesquels $Q_1$ représente un groupe amino, guanidino, amidino, $(C_1$-$C_4)$alkoxycarbonylamino, $(C_1$-$C_4)$alkylamino, di$(C_1$-$C_4)$alkylamino, $(C_1$-$C_4)$alkylamidino, guanidino, $(C_1$-$C_4)$alkylguanidino, amino$(C_1$-$C_4)$alkyl-N-$(C_1$-$C_4)$alkylamino.

5. Procédé de préparation des composés de formule I selon la revendication 1, caractérisé en ce que l'on fait réagir sur le nitrile de formule II :

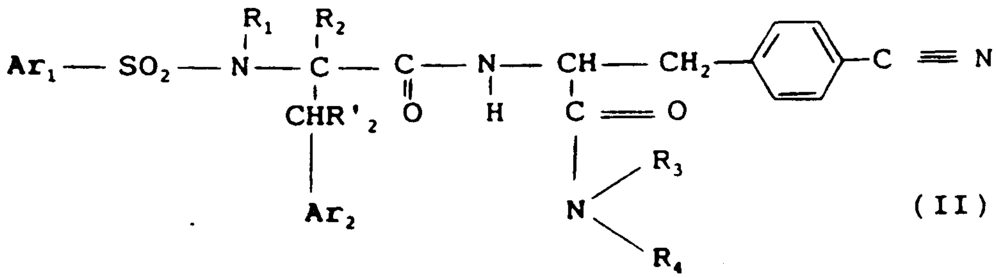

(II)

dans laquelle $Ar_1$, $R_1$, $R_2$, $Ar_2$, $R'_2$, $R_3$ et $R_4$ sont tels que définis à la revendication 1, sous forme d'un énantiomère pur ou d'un mélange d'isomères en proportions quelconques, un alcool ROH en milieu acide pour obtenir un imidoester intermédiaire, sur lequel on fait réagir l'amine de formule

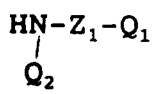

dans laquelle $Z_1$, $Q_1$, $Q_2$ ont la même signification que dans la formule I.

**6.** Composition pharmaceutique comprenant une dose thérapeutiquement active d'un composé de formule I selon l'une des revendications 1 à 3 sous forme d'énantiomères purs ou de mélange d'énantiomères ou un de leurs sels pharmaceutiquement acceptable en association avec au moins un excipient.

**7.** Composé selon la revendication 1 de formule I dans laquelle

$Ar_1$ représente naphtyle ou phényle éventuellement substitués par Cl, F, $(C_1$-$C_3)$alkyle ou $(C_1$-$C_3)$alkoxy;
$Ar_2$ représente phényle éventuellement substitués par Cl, F, $(C_1$-$C_3)$alkyle ou $(C_1$-$C_3)$alkoxy;
$R_1$, $R_2$ et $R'_2$ représentent indépendamment l'un de l'autre H, $(C_1$-$C_4)$alkyle ou $R_1$ ne représente rien et N est lié à $Ar_2$ et, éventuellement $R_2$ et $R'_2$ forment une double liaison,
ou $R_1$ ou $R_2$ est lié à $Ar_2$ et représente $(C_1$-$C_2)$ alkylène;
$R_3$ et $R_4$ identiques ou différents, représentent H, $(C_1$-$C_4)$alkyle ou forment avec l'atome d'azote auquel ils sont liés un hétérocycle saturé choisi parmi pyrrolidine, et pipéridine;
$Z_1$ représente $(C_1$-$C_{12})$alkylène interrompu ou prolongé éventuellement par $(C_5$-$C_7)$cycloalkyle ou phényle;
$Q_1$ représente méthyle, amino, $(C_1$-$C_4)$alkoxycarbonylamino, $(C_1$-$C_4)$alkylamino, di$(C_1$-$C_4)$alkylamino, pyrrolidinyle, pipéridino, morpholino, pipérazinyle, $(C_1$-$C_4)$alkyl-4 pipérazinyle, amidino, $(C_1$-$C_4)$alkylamidino, guanidino, $(C_1$-$C_4)$alkylguanidino, pyridyle, imidazolyle, pyrimidinyle, hydroxy, $(C_1$-$C_7)$alkoxy, $(C_2$-$C_8)$alkoxycarbonyle, amino$(C_1$-$C_4)$alkyl-N-$(C_1$-$C_4)$alkylamino, carbamoyle ou phényle éventuellement substitué par Cl, F, $(C_1$ à $C_3)$alkyle ou $(C_1$ à $C_3)$alkoxy;
$Q_2$ représente H ou $(C_1$-$C_4)$alkyle;
$Q_3$ représente H ou $(C_1$-$C_4)$alkyle ou $Q_1$ et $Q_3$ sont liés pour former un hétérocycle et représentent ensemble $(C_2$-$C_3)$alkylène tandis que $Z_1$ ne représente rien, sous forme d'énantiomère pur ou de mélange d'énantiomères en proportions quelconques,
ainsi que son sel avec un acide.

## Claims

**1.** Compounds of formula I

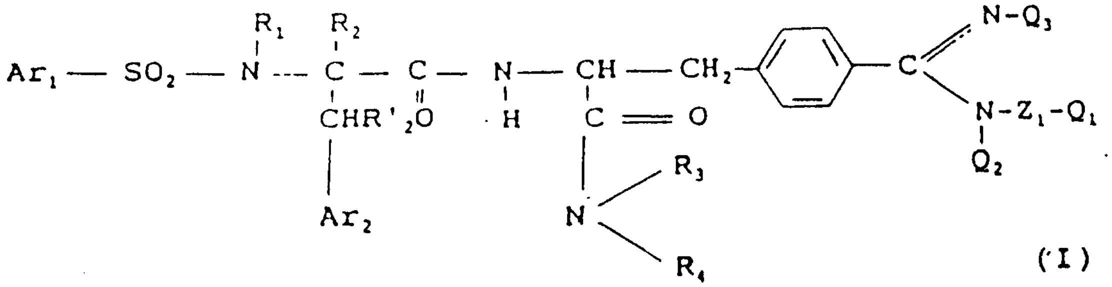

(I)

wherein

$Ar_1$ represents naphthyl, phenyl, quinolyl or isoquinolyl optionally substituted by Cl, F, $(C_{1-4})$alkyl, $(C_{1-4})$alkoxy, hydroxyl or $(C_{1-4})$dialkylamino,

$Ar_2$ denotes phenyl or thienyl, optionally substituted by Cl, F, $(C_{1-4})$alkyl, $(C_{1-4})$alkoxy or hydroxyl;

$R_1$, $R_2$ and $R'_2$ independently of one another denote H, $(C_{1-4})$alkyl or $R_1$ represents nothing and N is linked to $Ar_2$ and, optionally, $R_2$ and $R'_2$ form a double bond, or $R_1$ or $R_2$ is linked to $Ar_2$ and denotes $(C_{1-3})$alkylene;

$R_3$ and $R_4$, which may be identical or different, represent H, $(C_{1-4})$alkyl or, together with the nitrogen atom to which they are linked, form a $(C_{5-7})$ saturated heterocyclic group selected from pyrrolidine, piperidine and hexahydroazepine;

$Z_1$ represents $(C_{1-12})$alkylene optionally interrupted or prolonged by $(C_{5-7})$cycloalkyl or phenyl;

$Q_1$ denotes methyl, amino, $(C_{1-4})$alkoxycarbonylamino, $(C_{1-4})$alkylamino, di$(C_{1-4})$alkylamino, pyrrolidinyl, piperidino, morpholino, piperazinyl, $(C_{1-4})$alkyl-4-piperazinyl, amidino, $(C_{1-4})$alkylamidino, guanidino, $(C_{1-4})$alkylguanidino, pyridyl, imidazolyl, pyrimidinyl, indolyl, hydroxy, $(C_{1-4})$alkoxy, $(C_{2-8})$alkoxycarbonyl, amino $(C_{1-4})$alkyl-N-$(C_{1-4})$alkylamino, carbamoyl or phenyl optionally substituted by Cl, F, $(C_{1-4})$alkyl, $(C_{1-4})$alkoxy or hydroxyl;

$Q_2$ denotes H or $(C_{1-4})$alkyl;

$Q_3$ denotes H or $(C_{1-4})$alkyl or $Q_1$ and $Q_3$ are linked to form a heterocyclic group and together represent $(C_{2-3})$alkylene, whereas $Z_1$ represents nothing, in the form of pure enantiomers or mixtures thereof in any desired proportions,

and the salts thereof with acids.

2. Compounds according to claim 1 of formula I wherein $NR_3R_4$ denotes pyrrolidinyl.

3. Compounds according to one of claims 1 and 2 of formula I wherein $Z_1$ denotes $(C_{4-9})$alkylene and $Q_1$ comprises a nitrogen atom fixed to $Z_1$.

4. Compounds according to clam 3, wherein $Q_1$ denotes an amino, guanidino, amidino, $(C_{1-4})$alkoxycarbonylamino, $(C_{1-4})$alkylamino, di$(C_{1-4})$alkylamino, $(C_{1-4})$alkylamidino, guanidino, $(C_{1-4})$alkylguanidino, amino $(C_{1-4})$alkyl-N-$(C_{1-4})$alkylamino.

5. Process for preparing compounds of formula I according to claim 1, characterised in that the nitrile of formula II:

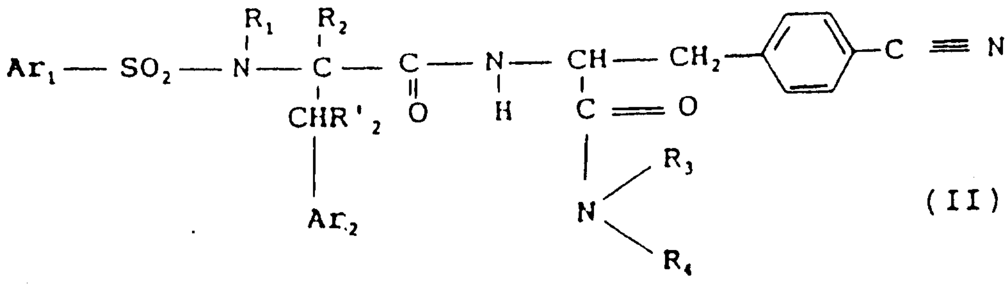

(II)

wherein $Ar_1$, $R_1$, $R_2$, $Ar_2$, $R'_2$, $R_3$ and $R_4$ are as defined in claim 1, in the form of a pure enantiomer or a mixture of isomers in any desired proportions, is reacted with an alcohol ROH in an acid medium to obtain an intermediate imidoester, which is reacted with the amine of formula

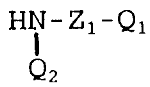

wherein $Z_1$, $Q_1$, $Q_2$ have the same meanings as in formula I.

6. Pharmaceutical composition containing a therapeutically active dose of a compound of formula I according to one of claims 1 to 3, in the form of pure enantiomers or a mixture of enantiomers or a pharmaceutically acceptable salt thereof combined with at least one excipient.

7. Compounds according to claim 1 of formula I wherein

$Ar_1$ represents naphthyl or phenyl optionally substituted by Cl, F, $(C_{1-3})$alkyl or $(C_{1-3})$alkoxy;

$Ar_2$ denotes phenyl optionally substituted by Cl, F, $(C_{1-3})$alkyl or $(C_{1-3})$alkoxy;

$R_1$, $R_2$ and $R'_2$ independently of one another denote H, $(C_{1-4})$alkyl or $R_1$ represents nothing and N is linked to $Ar_2$ and, optionally, $R_2$ and $R'_2$ form a double bond, or $R_1$ or $R_2$ is linked to $Ar_2$ and denotes $(C_{1-2})$alkylene;

$R_3$ and $R_4$, which may be identical or different, represent H, $(C_{1-4})$alkyl or, together with the nitrogen atom to which they are linked, form a saturated heterocyclic group selected from pyrrolidine and piperidine;

$Z_1$ represents $(C_{1-12})$alkylene optionally interrupted or prolonged by $(C_{5-7})$cycloalkyl or phenyl;

$Q_1$ denotes methyl, amino, $(C_{1-4})$alkoxycarbonylamino, $(C_{1-4})$alkylamino, di$(C_{1-4})$alkylamino, pyrrolidinyl, piperidino, morpholino, piperazinyl, $(C_{1-4})$alkyl-4-piperazinyl, amidino, $(C_{1-4})$alkylamidino, guanidino, $(C_{1-4})$alkylguanidino, pyridyl, imidazolyl, pyrimidinyl, hydroxy, $(C_{1-7})$alkoxy, $(C_{2-8})$alkoxycarbonyl, amino $(C_{1-4})$alkyl-N-$(C_{1-4})$alkylamino, carbamoyl or phenyl optionally substituted by Cl, F, $(C_{1-3})$alkyl or $(C_{1-3})$alkoxy;

$Q_2$ denotes H or $(C_{1-4})$alkyl;

$Q_3$ denotes H or $(C_{1-4})$ alkyl or $Q_1$ and $Q_3$ are linked to form a heterocyclic group and together represent $(C_{2-3})$alkylene, whereas $Z_1$ represents nothing, in the form of pure enantiomers or a mixture thereof in any desired proportions,

and the salt thereof with an acid.

**Patentansprüche**

1. Verbindungen der Formel I

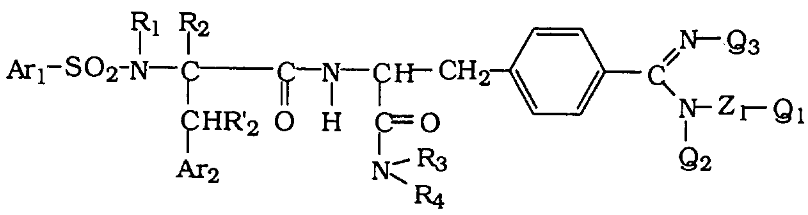

(I)

in der

$Ar_1$ Naphthyl, Phenyl, Chinolyl oder Isochinolyl darstellt, die gegebenenfalls durch Cl, F, $(C_1-C_4)$-Alkyl, $(C_1-C_4)$-Alkoxy, Hydroxy oder $(C_1-C_4)$-Dialkylamino substituiert sind,

$Ar_2$ Phenyl oder Thienyl darstellt, die gegebenenfalls durch Cl, F, $(C_1-C_4)$-Alkyl, $(C_1-C_4)$-Alkoxy oder Hydroxy substituiert sind;

$R_1$, $R_2$ und $R'_2$ unabhängig voneinander H, $(C_1-C_4)$-Alkyl darstellen oder $R_1$ nichts bedeutet und N an $Ar_2$ gebunden ist, und gegebenenfalls $R_2$ und $R'_2$ eine Doppelbindung bilden,
oder $R_1$ oder $R_2$ an $Ar_2$ gebunden sind und $(C_1-C_3)$-Alkylen bedeuten;

$R_3$ und $R_4$, die gleichartig oder verschieden sind, H, $(C_1-C_4)$-Alkyl darstellen oder zusammen mit dem Stickstoffatom, an das sie gebunden sind, einen gesättigten $(C_5-C_7)$-Heterocyclus darstellen, ausgewählt aus Pyrrolidin, Piperidin und Hexahydroazepin;

$Z_1$ $(C_1-C_{12})$-Alkylen darstellt, welches gegebenenfalls durch $(C_5-C_7)$-Cycloalkyl oder Phenyl unterbrochen oder verlängert ist;

$Q_1$ Methyl, Amino, $(C_1-C_4)$-Alkoxycarbonylamino, $(C_1-C_4)$-Alkylamino, Di-$(C_1-C_4)$-alkylamino, Pyrrolidinyl, Piperidino, Morpholino, Piperazinyl, $(C_1-C_4)$-Alkyl-4-piperazinyl, Amidino, $(C_1-C_4)$-Alkylamidino, Guanidino, $(C_1-C_4)$-Alkylguanidino, Pyridyl, Imidazolyl, Pyrimidinyl, Indolyl, Hydroxy, $(C_1-C_4)$-Alkoxy, $(C_2-C_8)$-Alkoxycarbonyl, Amino-$(C_1-C_4)$-alkyl-N-$(C_1-C_4)$-alkylamino, Carbamoyl oder Phenyl, welches gegebenenfalls durch Cl, F, $(C_1-C_4)$-Alkyl, $(C_1-C_4)$-Alkoxy oder Hydroxy substituiert ist, bedeutet;

$Q_2$ H oder $(C_1-C_4)$-Alkyl darstellt;

$Q_3$ H oder $(C_1-C_4)$-Alkyl darstellt oder $Q_1$ und $Q_3$ verbunden sind unter Bildung eines Heterocyclus und gemeinsam $(C_2-C_3)$-Alkylen darstellen, während $Z_1$ nichts bedeutet, in Form der reinen Enantiomeren oder deren Mischungen in beliebigen Verhältnissen,
sowie deren Salze mit Säuren.

**2.** Verbindungen nach Anspruch 1 der Formel I, in der $NR_3R_4$ Pyrrolidinyl bedeutet.

**3.** Verbindungen nach einem der Ansprüche 1 und 2 der Formel I, in der $Z_1$ $C_4$-$C_9$-Alkylen darstellt und $Q_1$ ein an $Z_1$ gebundenes Stickstoffatom trägt.

**4.** Verbindungen nach Anspruch 3, in denen $Q_1$ eine Aminogruppe, Guanidinogruppe, Amidinogruppe, $(C_1-C_4)$-Alkoxycarbonylaminogruppe, $(C_1-C_4)$-Alkylaminogruppe, Di-$(C_1-C_4)$-alkylaminogruppe, $(C_1-C_4)$-Alkylamidinogruppe, Guanidinogruppe, $(C_1-C_4)$-Alkylguanidinogruppe oder Amino-$(C_1-C_4)$-alkyl-N-$(C_1-C_4)$-alkylaminogruppe bedeutet.

**5.** Verfahren zur Herstellung der Verbindungen der Formel I nach Anspruch 1, dadurch gekennzeichnet, daß man das Nitril der Formel II:

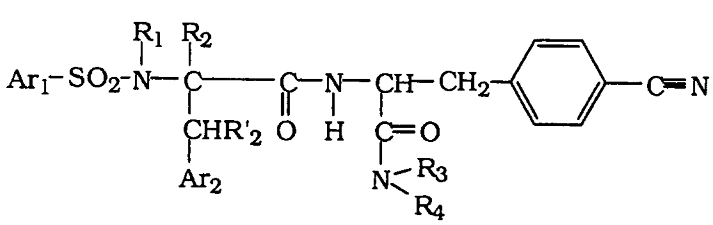

(II)

in der $Ar_1$, $R_1$, $R_2$, $Ar_2$, $R'_2$, $R_3$ und $R_4$ die in Anspruch 1 angegebenen Bedeutungen besitzen, in Form des reinen Enantiomeren oder einer Mischung der Isomeren in beliebigen Verhältnissen mit einem Alkohol ROH in saurem

Medium umsetzt zur Bildung eines Imidoester-Zwischenprodukts, welches man mit dem Amin der Formel

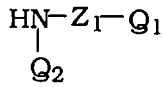

in der $Z_1$, $Q_1$ und $Q_2$ die bezüglich der Formel I angegebenen Bedeutungen besitzen, umsetzt.

**6.** Pharmazeutische Zubereitung enthaltend eine therapeutisch wirksame Dosis einer Verbindung der Formel I nach einem der Ansprüche 1 bis 3 in Form der reinen Enantiomeren oder einer Enantiomerenmischung oder deren pharmazeutisch annehmbarer Salze in Kombination mit mindestens einem Trägermaterial.

**7.** Verbindung nach Anspruch 1 der Formel I, in der

$Ar_1$ Naphthyl oder Phenyl darstellt, die gegebenenfalls durch Cl, F, ($C_1$-$C_3$)-Alkyl oder ($C_1$-$C_3$)-Alkoxy substituiert sind;
$Ar_2$ Phenyl darstellt, welches gegebenenfalls durch Cl, F, ($C_1$-$C_3$)-Alkyl oder ($C_1$-$C_3$)-Alkoxy substituiert sind;
$R_1$, $R_2$ und $R'_2$ unabhängig voneinander H oder ($C_1$-$C_4$)-Alkyl bedeuten oder $R_1$ nichts darstellt und N an $Ar_2$ gebunden ist, und gegebenenfalls $R_2$ und $R'_2$ eine Doppelbindung bilden,
oder $R_1$ oder $R_2$ an $Ar_2$ gebunden sind und ($C_1$-$C_3$)-Alkylen darstellen;
$R_3$ und $R_4$, die gleichartig oder verschieden sind, H, ($C_1$-$C_4$)-Alkyl darstellen oder zusammen mit dem Stickstoffatom, an das sie gebunden sind, einen gesättigten Heterocyclus ausgewählt aus Pyrrolidin und Piperidin bilden;
$Z_1$ ($C_1$-$C_{12}$)-Alkylen darstellt, das gegebenenfalls durch ($C_5$-$C_7$)-Cycloalkyl oder Phenyl unterbrochen oder verlängert ist;
$Q_1$ Methyl, Amino, ($C_1$-$C_4$)-Alkoxycarbonylamino, ($C_1$-$C_4$)-Alkylamino, Di-($C_1$-$C_4$)-alkylamino, Pyrrolidinyl, Piperidino, Morpholino, Piperazinyl, ($C_1$-$C_4$)-Alkyl-4-piperazinyl, Amidino, ($C_1$-$C_4$)-Alkylamidino, Guanidino, ($C_1$-$C_4$)-Alkylguanidino, Pyridyl, Imidazolyl, Pyrimidinyl, Hydroxy, ($C_1$-$C_7$)-Alkoxy, ($C_2$-$C_8$)-Alkoxycarbonyl, Amino-($C_1$-$C_4$)-alkyl-N-($C_1$-$C_4$)-alkylamino, Carbamoyl oder Phenyl, das gegebenenfalls durch Cl, F, ($C_1$-$C_3$)-Alkyl oder ($C_1$-$C_3$)-Alkoxy substituiert ist, bedeutet;
$Q_2$ H oder ($C_1$-$C_4$)-Alkyl darstellt;
$Q_3$ H oder ($C_1$-$C_4$)-Alkyl darstellt oder $Q_1$ und $Q_3$ verbunden sind unter Bildung eines Heterocyclus und gemeinsam ($C_2$-$C_3$)-Alkylen darstellen, während $Z_1$ nichts bedeutet, in Form des reinen Enantiomeren oder einer Mischungen von Enantiomeren in beliebigen Verhältnissen
sowie deren Salze mit einer Säure.